(19) Europäisches Patentamt
European Patent Office
Office européen des brevets

(11) Veröffentlichungsnummer: **0 229 623**
**A2**

(12) **EUROPÄISCHE PATENTANMELDUNG**

(21) Anmeldenummer: **87100075.8**

(22) Anmeldetag: **07.01.87**

(51) Int. Cl.$^3$: **C 07 D 295/08**
C 07 D 217/02, C 07 D 401/12
C 07 D 215/22, C 07 D 215/20
C 07 D 211/22, A 61 K 31/495
A 61 K 31/47, A 61 K 31/44
C 07 D 215/26, C 07 D 217/04
//(C07D401/12,243:00,217:00),
(C07D401/12,217:00,211:00)

(30) Priorität: **09.01.86 DE 3600390**

(43) Veröffentlichungstag der Anmeldung:
**22.07.87 Patentblatt 87/30**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**

(72) Erfinder: **Urbach, Hansjörg, Dr.**
**Le Lavandou-Strasse 41**
**D-6242 Kronberg/Taunus(DE)**

(72) Erfinder: **Bartmann, Wilhelm, Dr.**
**Am Dachsbau 5**
**D-6232 Bad Soden am Taunus(DE)**

(72) Erfinder: **Kaiser, Joachim, Dr.**
**Fichtestrasse 12**
**D-6000 Frankfurt am Main(DE)**

(54) **Diarylalkyl-substituierte Alkylamine, Verfahren zu ihrer Herstellung, ihre Verwendung sowie sie enthaltende Arzneimittel.**

(57) Beschrieben werden Verbindungen I

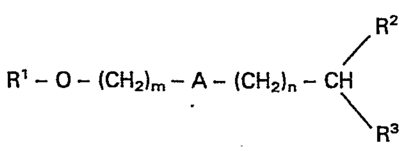

(I)

mit $R^1$ gleich Cycloalkyl, Alkenyl, Cycloalkenyl, Phenyl,

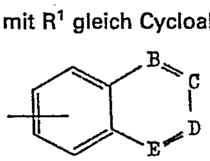

oder

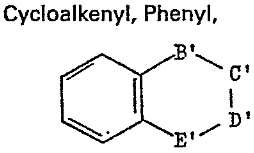

mit B, C, D, E gleich Methin oder Stickstoff und
B', C', D', E' Methylen, Carbonyl, Imino;
$R^2$ gleich Phenyl oder Phenylalkyl;
A gleich verschiedene Aminreste;
m gleich 2, 3 oder 4;
n gleich 1, 2, 3 oder 4;
beschrieben werden auch Salze dieser Verbindungen I. Verbindungen I und die Salze sind Calcium-Antagonisten.

EP 0 229 623 A2

0229623

Diarylalkyl-substituierte Alkylamine, Verfahren zu ihrer Herstellung, ihre Verwendung sowie sie enthaltende Arzneimittel

Die Erfindung betrifft Diarylalkyl-substituierte Alkylamine, Verfahren zu ihrer Herstellung, ihre Verwendung als Heilmittel und sie enthaltende Arzneimittel, sowie Zwischenprodukte zu ihrer Herstellung.

Zahlreiche Diarylbutylpiperidin- und Diarylbutylpiperazinderivate werden aufgrund ihrer Wirkung als Antagonisten des Dopamins therapeutisch als Neuroleptika genutzt. Strukturell verwandte Verbindungen aus der Reihe der Benzhydrylpiperazin- und der Diarylbutylpiperazinderivate wirken als Hemmstoffe des Einstroms von Calcium-Ionen in Zellen, wie z.B. Cinnarizin, Flunarizin und Lidoflazin. Sie finden Anwendung als Therapeutika zur Behandlung von Herz-Kreislauf- und cerebrovaskulären Erkrankungen.

N-Arylpiperazinalkanamide mit Diarylbutylsubstituenten an Piperazinsystemen werden in der Europäischen Patentanmeldung 68544 beschrieben: sie verbessern die Durchblutung des Herzens und schützen es vor den Folgen einer Ischämie, Anoxie oder Hypoxie.

Aus der US-Patentschrift 3634432 sind N-(ω-Alkoxyalkyl)-3- und -4-benzhydrylpiperidine bekannt, welche als Magensäuresekretionshemmer Verwendung finden. Sie unterscheiden sich von den erfindungsgemäßen Verbindungen durch den Substituenten an der veretherten Hydroxygruppe $X_1$ (siehe Beispiele 12 und 13), der niederes Alkyl, Aralkyl oder 2-Tetrahydropyranyl sein kann (siehe Spalte 2, Zeilen 19-23), jedoch in keinem Falle die Bedeutung des entsprechenden Substituenten $R^1$ nach der Erfindung haben kann. Ebensowenig ist an eine calciumantagonistische Wirkung gedacht worden.

Die spanische Patentschrift 504202 beschreibt Benzhydrylpiperazin-Derivate, in denen aber stets die Benzhydrylgruppe unmittelbar an den einen Stickstoff des Piperazins gebunden ist, nicht jedoch über eine $(CH_2)_n$-Brücke. Darüber hinaus zeigen diese Verbindungen lediglich eine vasodilatorische Wirkung, nicht aber eine calciumantagonistische Wirkung.

Es war daher überraschend, daß die Verbindungen der vorliegenden Erfindung den Einstrom von Calcium-Ionen in Zellen in ungewöhnlich starkem Ausmaß behindern. Sie eignen sich daher als Therapeutika zur Behandlung verschiedener Krankheiten, insbesondere des Herz-Kreislauf-Systems und der cerebralen Gefäße.

Die vorliegende Erfindung ist gerichtet auf Verbindungen der Formel I,

$$R^1 - O - (CH_2)_m - A - (CH_2)_n - CH\begin{smallmatrix}R^2\\ R^3\end{smallmatrix} \qquad (I)$$

die hervorragende calciumantagonistische Wirkung aufweisen und in welcher bedeuten:

$R^1$ $(C_3-C_8)$-Cycloalkyl, $(C_2-C_6)$-Alkenyl, geradkettig oder verzweigt, $(C_5-C_8)$-Cycloalkenyl,

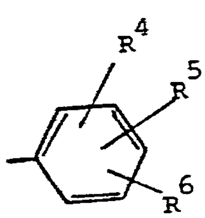 , 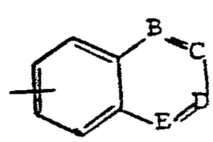 oder 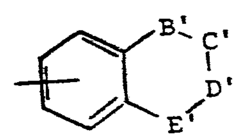

worin
$R^4$, $R^5$ und $R^6$ gleich oder verschieden unabhängig voneinander Wasserstoff, $(C_1-C_6)$-Alkyl, $(C_3-C_8)$-Cycloalkyl, Hydroxy, $(C_1-C_4)$-Alkoxy, $(C_1-C_4)$-Alkylthio, F, Cl, Br, J, Nitro, Cyano, Trifluormethyl, Formyl, Carboxyl, $(C_1-$

$C_6$)-Alkoxycarbonyl, ($C_1$-$C_6$)-Acyl, Carbamoyl, N-Mono- oder N,N-Di($C_1$-$C_6$)Alkylcarbamoyl, Sulfo, ($C_1$-$C_6$)-Alkoxysulfonyl, Sulfamoyl, N-Mono- oder N,N-Di-($C_1$-$C_6$)-Alkylsulfamoyl, ($C_1$-$C_6$)-Alkylsulfinyl, ($C_1$-$C_6$)-Alkylsulfonyl, Amino, unsubstituiert oder substituiert mit ein oder zwei gleich- oder verschiedenartigen ($C_1$-$C_6$)-Alkyl-, ($C_1$-$C_6$)-Acyl- oder Aryl-, vorzugsweise Phenylgruppen,

B, C, D und E gleich oder verschieden unabhängig voneinander Methin oder Stickstoff,

B', C', D' und E' gleich oder verschieden unabhängig voneinander Methylen, Carbonyl, Imino, unsubstituiert oder am Stickstoff substituiert durch ($C_1$-$C_6$)-Alkyl, ($C_1$-$C_6$)-Acyl oder Aryl-, vorzugsweise Phenyl,

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl , ($C_1$-$C_4$)-Alkoxy, F, Cl, Br, J, Cyano, Nitro oder Trifluormethyl substituiert ist,

A ein Amin 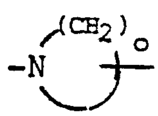 , 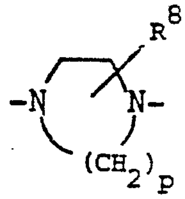 oder 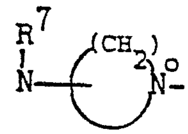

worin

$R^7$ Wasserstoff, ($C_1$-$C_6$)-Alkyl, Aryl, vorzugsweise Phenyl,

$R^8$ Wasserstoff, ($C_1$-$C_6$)-Alkyl, Formyl, ($C_1$-$C_6$)-Acyl, Carboxyl, ($C_1$-$C_6$)-Alkoxycarbonyl, Carbamoyl, N-Mono- oder N,N -Di-($C_1$-$C_6$)-Alkylcarbamoyl,

o 3, 4, 5, 6 oder 7,

p 2 oder 3,

m 2, 3 oder 4,

n 1, 2, 3 oder 4 bedeuten,

sowie die Salze der Verbindungen der Formel I mit physiologisch verträglichen Säuren.

Bevorzugt werden Verbindungen der Formel I, in welcher mindestens einer der Reste und Indices folgende Bedeutung hat:

$R^1$ $(C_3-C_8)$-Cycloalkyl, 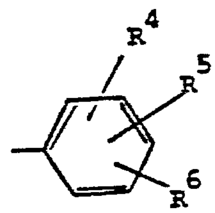 , 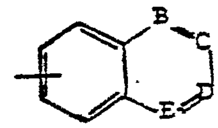

worin $R^4$ und $R^5$ gleich oder verschieden unabhängig voneinander Wasserstoff, $(C_1-C_6)$-Alkyl, $(C_1-C_4)$-Alkoxy, F, Cl, Br, J, Nitro, Cyano, Trifluormethyl, Formyl, Carboxyl, $(C_1-C_6)$-Alkoxycarbonyl, $(C_1-C_6)$-Acyl, Carbamoyl, N-Mono- oder N,N-Di-$(C_1-C_6)$-Alkylcarbamoyl, Sulfo, $(C_1-C_6)$-Alkoxysulfonyl, Sulfamoyl, N-Mono- oder N,N-Di-$(C_1-C_6)$-Alkylsulfamoyl, $(C_1-C_6)$-Alkylsulfinyl, $(C_1-C_6)$-Alkylsulfonyl,

$R^6$ Wasserstoff,

B, C, D und E gleich oder verschieden unabhängig voneinander Methin oder Stickstoff,

$R^2$ und $R^3$ gleich oder verschieden und unabhängig voneinander Phenyl, das unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,

A ein Amin

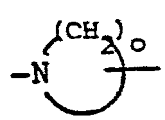 , 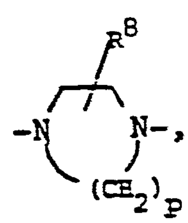 , 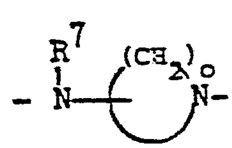

worin

$R^7$ Wasserstoff, Methyl, Ethyl,

$R^8$ Wasserstoff, Carboxyl, Carbamoyl,
o 4, 5 oder 6,
p 2 oder 3,
m 2, 3 oder 4,
n 1, 2, 3 oder 4 bedeuten,

sowie die Salze der Verbindungen der Formel I mit physiologisch verträglichen Säuren.

Besonders bevorzugt werden Verbindungen der Formel I, in welcher mindestens einer der Substituenten und Indices folgende Bedeutung hat:

$R^1$ $(C_5-C_7)$-Cycloalkyl, 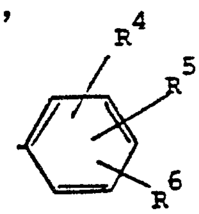 , 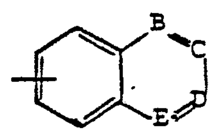

worin

$R^4$ Wasserstoff, Methyl, Ethyl, Propyl, Isopropyl, n-Butyl, s-Butyl, Isobutyl, tert. Butyl, Methoxy, Ethoxy, Fluor, Chlor, Brom, Nitro, Cyano, Trifluormethyl,
$R^5$ und $R^6$ Wasserstoff,

B, C, D und E gleich oder verschieden unabhängig voneinander Methin oder Stickstoff,

$R^2$ und $R^3$ gleich oder verschieden unabhängig voneinander Phenyl, das unsubstituiert oder durch einen, zwei oder drei Substituenten aus der Gruppe Methyl, Fluor, Chlor, Brom, Cyano, Nitro oder Trifluormethyl substituiert ist,

A ein Amin 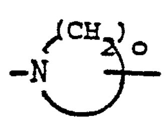 , 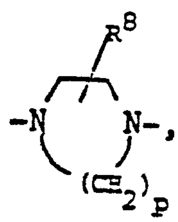 ,

worin

0229623

$R^8$ Wasserstoff, Carboxyl, Carbamoyl,
o 4, 5 oder 6,
p 2 oder 3,
m 2, 3 oder 4
n 2, 3 oder 4 bedeuten,
sowie die Salze der Verbindungen der Formel I mit physiologisch verträglichen Säuren.

Ganz besonders bevorzugt werden Verbindungen der Formel I, in welcher mindestens einer der Substituenten oder Indices folgende Bedeutung hat:

$R^1$ Cyclohexyl, Phenyl, das unsubstituiert oder durch Methyl, tert. Butyl, Methoxy, Fluor, Nitro, Cyano oder Trifluormethyl substituiert ist, Naphthyl, Chinolinyl oder Isochinolinyl,

$R^2$ und $R^3$ gleich oder verschieden unabhängig voneinander Phenyl, das unsubstituiert oder durch Fluor oder Trifluormethyl substituiert ist,

A ein Amin

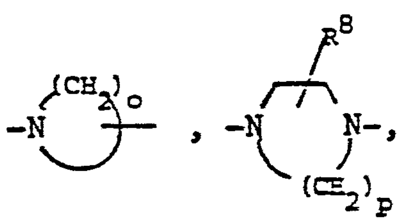

worin
$R^8$ Wasserstoff,
o 5,
p 2,
m 2,
n 3 bedeuten,
sowie die Salze der Verbindungen der Formel I mit physiologisch verträglichen Säuren.

Als solche Säuren kommen anorganische Säuren wie Salzsäure, Bromwasserstoffsäure, Jodwasserstoffsäure, Schwefelsäure, Phosphorsäure oder Salpetersäure oder organische Säuren wie

Weinsäure, Citronensäure, Äpfelsäure, Milchsäure, Maleinsäure, Fumarsäure, Malonsäure, Oxalsäure, Essigsäure, Propionsäure, Methansulfonsäure, Benzolsulfonsäure, p-Toluolsulfonsäure, Naphthalin-1,5-disulfonsäure oder Gluconsäure in Betracht.

Die Verbindungen der Formel I weisen zum Teil asymmetrische Kohlenstoffatome auf und können daher als Enantiomere und Diastereomere auftreten. Die Erfindung umfaßt sowohl die reinen Enantiomeren als auch die Racemate. Die Racemate können nach gebräuchlichen Methoden, zum Beispiel durch Salzbildung mit optisch aktiven Säuren wie Camphersulfonsäure oder Di-benzoylweinsäure, fraktionierte Kristallisation und anschließende Freisetzung der Basen aus ihren Salzen, oder durch Derivatisierung mit geeigneten optisch aktiven Reagenzien, Trennung der diastereomeren Derivate durch fraktionierte Kristallisation oder Chromatographie an Kieselgel oder Aluminiumoxid und Rückspaltung in die Enantiomeren aufgetrennt werden. Die Diastereomeren können nach gebräuchlichen Methoden, wie fraktionierte Kristallisation oder Chromatographie an Säulen, getrennt werden.

Die vorliegende Erfindung betrifft weiterhin ein Verfahren zur Herstellung von Verbindungen der Formel I, das dadurch gekennzeichnet ist, daß man

a) eine Verbindung der Formel II,

$$R^1 - O - (CH_2)_m - Y \qquad (II),$$

in welcher $R^1$ und m die gleiche Bedeutung wie in Formel I haben, und in welcher Y eine Abgangsgruppe, die nucleophil verdrängt werden kann, insbesondere ein Cl, Br, J-Atom oder einen Sulfonsäurerest, vorzugsweise einen Methansulfonyl-, Benzolsulfonyl-, p-Toluolsulfonyl- oder Trifluormethansulfonylrest bedeutet, mit einer Verbindung der Formel III,

$$Z - (CH_2)_n - CH \begin{matrix} \diagup R^2 \\ \diagdown R^3 \end{matrix} \qquad (III)$$

in welcher $R^2$, $R^3$ und n die gleiche Bedeutung wie in Formel I haben, und in welcher Z

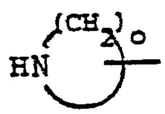 , 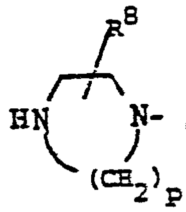 oder 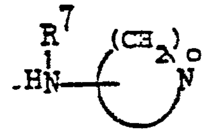 .

bedeutet, worin

$R^7$, $R^8$, o und p die gleiche Bedeutung wie in Formel I haben,

unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, Pyridin, 1,5-Diazabicyclo[5,4,0]undec-5-en oder 1,5-Diazabicyclo[4,3,0]non-5-en, sowie mit oder ohne Gegenwart eines Alkalihalogenids, vorzugsweise Natriumjodid oder Kaliumjodid, bei einer Temperatur zwischen 0 und 160°C, vorzugsweise zwischen 20 und 120°C, umsetzt,

oder daß man

b) eine Verbindung der Formel IV,

$$R^1 - O - (CH_2)_m - Z \qquad (IV)$$

in welcher $R^1$ und m die gleiche Bedeutung wie in Formel I und Z die gleiche Bedeutung wie in Formel III haben, mit einer Verbindung der Formel V,

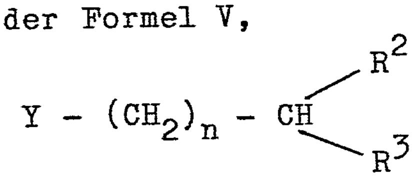 (V)

in welcher $R^2$, $R^3$ und n die gleiche Bedeutung wie in Formel I und Y die gleiche Bedeutung wie in Formel II haben,

unter den Bedingungen einer nucleophilen Substitution, wie unter a) beschrieben, umsetzt,

oder daß man

c) eine Verbindung der Formel VI,

$$R^1 - OH \qquad (VI)$$

in der $R^1$ die gleiche Bedeutung wie in Formel I besitzt, mit einer Verbindung der Formel VII,

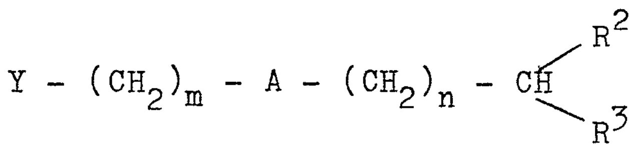

(VII)

in der $R^2$, $R^3$, A, m und n die gleiche Bedeutung wie in Formel I und Y die gleiche Bedeutung wie in Formel II haben, entweder in einem polaren aprotischen Lösungsmittel wie Acetonitril, Tetrahydrofuran, Dimethylsulfoxid, Dimethylformamid, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, vorzugsweise in Dimethylformamid oder Dimethylsulfoxid in Gegenwart von Natriumhydrid oder Natriumamid, bei einer Temperatur zwischen -40° und +100°C, vorzugsweise zwischen -20° und +50°C, oder in einem protischen oder aprotischen polaren organischen Lösungsmittel wie einem niederen Alkohol, beispielsweise Methanol, Ethanol, Isopropanol, oder einem niederen Keton, vorzugsweise Aceton, Methylethylketon oder Methylisobutylketon, oder in Dimethylformamid, in Gegenwart einer schwachen bis mittelstarken Base wie einem Alkali- oder Erdalkalimetallhydroxid oder -carbonat, vorzugsweise Natriumcarbonat oder Kaliumcarbonat, oder einem Amin wie beispielsweise Triethylamin, Pyridin, N-Ethyldiisopropylamin, 1,5-Diazabicyclo-[5,4,0]undec-5-en oder 1,5-Diazabicyclo[4,3,0]non-5-en, bei einer Temperatur zwischen 0° und 160°C, vorzugsweise zwischen 20° und 120°C, umsetzt, oder daß man

d) eine Verbindung der Formel XIII,

$$R^1 - W \qquad (XIII),$$

in der $R^1$ die gleiche Bedeutung wie in Formel I besitzt und in der W eine Abgangsgruppe, die nucleophil verdrängt werden kann, insbesondere ein Fluor-, Chlor-, Brom- oder Iodatom, eine Nitro-, Hydroxy-, Alkoxy- oder Trialkylammoniogruppe oder einen Sulfonsäurerest, vorzugsweise ein Fluor- oder Chloratom, eine Nitrogruppe oder einen Methansulfonyl-, Benzolsulfonyl-, p-Toluolsulfonyl- oder Trifluormethansulfonylrest bedeutet, mit einer Verbindung der Formel XIV,

$$HO-(CH_2)_m-A-(CH_2)_n-CH\begin{matrix} R^2 \\ R^3 \end{matrix} \qquad (XIV)$$

in der $R^2$, $R^3$, A, m und n die gleiche Bedeutung wie in Formel I besitzen, unter Bedingungen einer nucleophilen Substitution, beispielsweise ohne Lösungsmittel oder in einem wäßrigen Lösungsmittel, vorzugsweise in einem polaren organischen Lösungsmittel wie einem Alkohol, bevorzugt Methanol, Ethanol, Propanol oder Isopropanol, oder einem Keton, bevorzugt Aceton, Methylethylketon oder Methylisobutylketon, oder einem Ether, bevorzugt Diethylether, tert. Butylmethylether, Dimethoxyethan, Tetrahydrofuran oder Dioxan, oder einem halogenierten Kohlenwasserstoff, bevorzugt Dichlormethan, Chloroform oder 1,2-Dichlorethan, oder in Acetonitril, Dimethylformamid, Dimethylsulfoxid oder Sulfolan, oder in einem Kohlenwasserstoff, bevorzugt Benzol oder Toluol, oder in einem gemischten wäßrig-organischen Lösungsmittelsystem unter Zusatz eines Phasentransferkatalysators, mit oder

ohne Gegenwart einer Hilfsbase zum Abfangen der sich bildenden Säure, vorzugsweise in Gegenwart von Kaliumcarbonat, Natriumcarbonat, Triethylamin, Pyridin, Lithiumdiisopropylamid, n-Butyllithium, Natriumhydrid, Kaliumhydrid, Natriumamid, 1,5-Diazabicyclo-[5.4.0]undec-5-en oder 1,5-Diazabicyclo[4.3.0]-non-5-en, sowie mit oder ohne Gegenwart von Kupferpulver bei einer Temperatur zwischen -80 und +200°C, vorzugsweise zwischen -30° und +120°C, umsetzt.

Die Verbindungen der Formel II sind literaturbekannt bzw. lassen sich unter analogen Bedingungen aus Verbindungen der Formel VI durch Umsetzung mit $\alpha,\omega$-Dihalogenalkanen oder $\omega$-Halogenalkylsulfonaten herstellen.

Die Verbindungen der Formel III, mit Z gleich

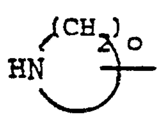 , 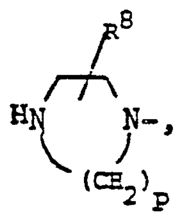 , oder 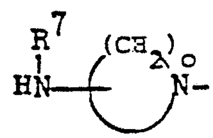

worin $R^7$, $R^8$, o und p die gleiche Bedeutung wie in Formel I haben, lassen sich in an sich bekannter Weise aus Verbindungen der Formel VIII,

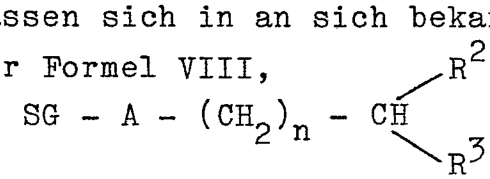 (VIII)

worin $R^2$, $R^3$, A und n die gleiche Bedeutung wie in Formel I haben und worin SG eine geeignete Schutzgruppe, wie z. B. eine Carbamat-, Amid-, Alkyl- oder Benzylgruppe, vorzugsweise eine Formyl-, Ethoxycarbonyl-, Benzyl- oder Tritylgruppe bedeutet, durch Abspaltung der Schutzgruppe unter literaturbekannten Bedingungen, z.B. saure oder alkalische Spaltung oder durch Hydrogenolyse, herstellen.

Die Verbindungen der Formel III, mit Z gleich

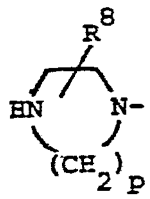

oder

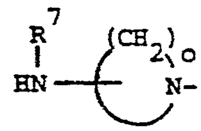

worin $R^7$, $R^8$, o und p die gleiche Bedeutung wie in Formel I haben, lassen sich außerdem herstellen durch Umsetzung von Verbindungen der Formel V mit Aminen der Formel Z - H, wobei Z eine der obengenannten Gruppen ist, unter den Bedingungen einer nucleophilen Substitution, wie sie unter der Verfahrensvariante a) beschrieben sind,

oder

durch Umsetzung von Verbindungen der Formel V mit geschützten Aminen

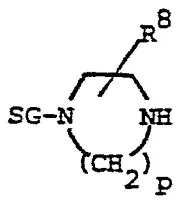

oder

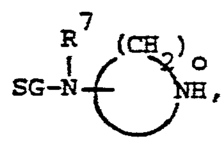

worin $R^7$, $R^8$, o und p die gleiche Bedeutung wie in Formel I und SG die gleiche Bedeutung wie in Formel VIII haben, unter den Bedingungen einer nucleophilen Substitution, wie sie unter der Verfahrensvariante a) beschrieben sind, mit anschließender Abspaltung der Schutzgruppe unter üblichen Bedingungen, z.B. durch saure oder alkalische Spaltung oder durch Hydrogenolyse.

Die Erfindung betrifft ebenfalls Verbindungen der Formel III

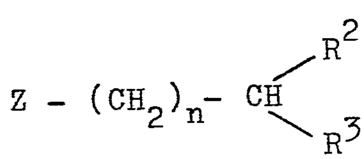 (III),

in welcher bedeuten:

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl-, ($C_1$-$C_4$)-

Alkoxy, F, Cl, Br, J, Cyano, Nitro oder Trifluormethyl substituiert ist,

Z 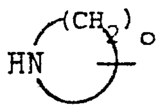 oder 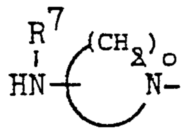 und

n 1, 2, 3 oder 4,

jedoch mit Ausnahme der Verbindung, in welcher gleichzeitig $R^2=R^3$=Phenyl, n=1 und Z= 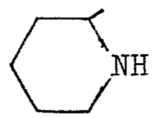 ist.

Die Verbindungen der Formel III, in denen Z

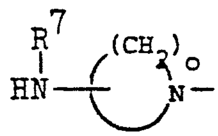 ist,

worin o die gleiche Bedeutung wie in Formel I hat und $R^7$ Methyl ist, lassen sich bevorzugt durch Reduktion mit geeigneten Reduktionsmitteln, vorzugsweise Lithiumaluminiumhydrid, aus Verbindungen der gleichen Formel, worin $R^7$ einen Alkoxycarbonylrest bedeutet, herstellen.

Die Verbindungen der Formel IV lassen sich aus den Verbindungen der Formel II nach analogen Verfahren, wie sie bereits für die Darstellung der Verbindungen der Formel III aus den Verbindungen der Formel V beschrieben sind, herstellen,

oder

aus Verbindungen der Formel IX

$$R^1 - O - (CH_2)_m - A - SG \quad (IX)$$

worin $R^1$, A und m die gleiche Bedeutung wie in Formel I und SG die gleiche Bedeutung wie in Formel VIII besitzen,

durch Abspaltung der Schutzgruppe unter den üblichen Bedingungen, z. B. durch saure oder alkalische Spaltung oder durch Hydrogenolyse,

oder

aus Verbindungen der Formel VI durch Umsetzung mit Verbindungen der Formel X

$$Y - (CH_2)_m - A - SG \quad (X)$$

worin A und m die gleiche Bedeutung wie in Formel I, Y die gleiche Bedeutung wie in Formel II und SG die gleiche Bedeutung wie in Formel VIII besitzen, unter den Bedingungen einer Alkylierungsreaktion, wie sie unter der Verfahrensvariante c) beschrieben sind, gefolgt von einer Abspaltung der Schutzgruppe unter den üblichen Bedingungen.

Die Verbindungen der Formel V sind großenteils literaturbekannt bzw. können in analoger Weise hergestellt werden.

Die Verbindungen der Formel VI sind literaturbekannt und zumeist käuflich.

Die Erfindung schließt weiterhin die Verbindungen der Formel VII ein

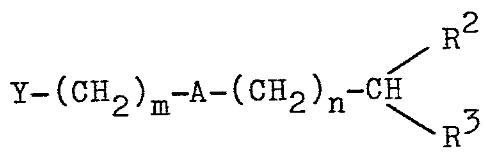

(VII),

in welcher bedeuten:

$R^2$ und $R^3$ gleich oder verschieden und unabhängig voneinander Phenyl, Phenyl-$(C_1-C_4)$-alkyl, wobei der Phenylring jeweils unsubstituiert oder durch einen, zwei oder drei Substituenten aus der Gruppe $(C_1-C_4)$-Alkyl, $(C_1-C_4)$-Alkoxy, F, Cl, Br, J, Cyano, Nitro oder Trifluormethyl substituiert ist,

n 1, 2, 3 oder 4,

A ein Amin 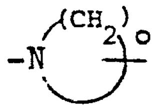 , 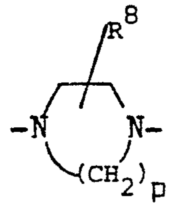 oder 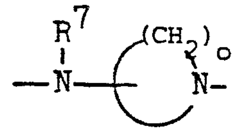

worin

$R^7$ Wasserstoff, $(C_1-C_6)$-Alkyl, Aryl, vorzugsweise Phenyl,

$R^8$ Wasserstoff, $(C_1-C_6)$-Alkyl, Formyl, $(C_1-C_6)$-Acyl, Carboxyl, $(C_1-C_6)$-Alkoxycarbonyl, Carbamoyl, N-Mono- oder N,N-Di-$(C_1-C_6)$-Alkylcarbamoyl,

o 3, 4, 5, 6 oder 7,

p 2 oder 3,

m 2, 3 oder 4,

Y eine Abgangsgruppe, die nucleophil verdrängt werden kann,

jedoch mit der Ausnahme der Verbindung, in welcher gleichzeitig

$R^2 = R^3$ = 4-Fluorphenyl,

n = 3

A = 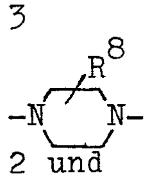 mit $R^8$ = H

m = 2 und

Y = Cl sind

und mit Ausnahme der Verbindung, in welcher gleichzeitig

$R^2$=$R^3$=Phenyl, n=1, A= 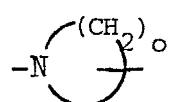 mit o=5, m=2 und Y=Cl sind.

Die Verbindungen der Formel VII lassen sich herstellen aus den Verbindungen der Formel III durch Umsetzung mit $\alpha,\omega$-Dihalogenalkanen oder $\omega$-Halogenalkylsulfonaten unter den Bedingungen einer nucleophilen Substitution, wie sie unter der Verfahrensvariante a) beschrieben sind

oder

aus den Verbindungen der Formel V durch Umsetzung mit einer Verbindung der Formel XI,

$Y - (CH_2)_m - Z$ (XI)

worin m die gleiche Bedeutung wie in Formel I, Y die gleiche Bedeutung wie in Formel II und Z die gleiche Bedeutung wie in Formel III besitzen, unter den Bedingungen einer nucleophilen Substitution, wie sie unter der Verfahrensvariante a) beschrieben sind,

oder

aus den Verbindungen der Formel V durch Umsetzung mit einer Verbindung der Formel XII,

$HO - (CH_2)_m - Z$ (XII)

worin m die gleiche Bedeutung wie in Formel I und Z die gleiche Bedeutung wie in Formel III besitzen, unter den Bedingungen einer nucleophilen Substitution, wie sie unter der Verfahrensvariante a) beschrieben sind, zu Verbindungen der Formel XIV, und anschließende Überführung der Hydroxyfunktion in die Abgangsgruppe Y nach gängigen Methoden.

Die Verbindungen der Formel VIII werden mit Hilfe der allgemeinen Verfahren der Schutzgruppenchemie aus den Verbindungen der Formel III hergestellt

oder

aus den Verbindungen der Formel V durch Umsetzung mit geschützten Aminen, wie bereits bei der Herstellung von Verbindungen der Formel III aus solchen der Formel V beschrieben.

Die Verbindungen der Formel IX werden mit Hilfe der allgemeinen Verfahren der Schutzgruppenchemie aus den Verbindungen der Formel IV hergestellt,

oder

aus den Verbindungen der Formel VI durch Umsetzung mit Verbindungen der Formel X, wie bereits bei der Herstellung von Verbindungen der Formel IV aus solchen der Formel VI beschrieben.

Die Verbindungen der Formeln X, XI, XII und XIII sind bekannt bzw. nach einfachen Verfahren aus käuflichen Ausgangsmaterialien zugänglich.

Die erfindungsgemäßen Verbindungen der Formel I weisen biologische Wirkungen, insbesondere calciumantagonistische Wirkungen auf, und besitzen daher wertvolle Eigenschaften zur Behandlung aller Krankheitszustände, die auf einer Störung im Calciumhaushalt beruhen: insbesondere sind sie als blutdrucksenkende Mittel, antianginös wirkende Mittel und zur Verbesserung der cerebralen Gefäßdurchblutung geeignet.

Ihre calciumantagonistische Wirksamkeit kann in dem biochemischen Testmodell der Verdrängung von tritiummarkierten Nitrendipin gezeigt werden. Diese Prüfung führten wir in einer aus dem Cortex des Rattenhirns gewonnenen und mehrfach gewaschenen Membranpräparation aus, wobei wir im wesentlichen die von R. J. Gould et al. (Proc. Natl. Acad. Sci. USA <u>79</u>, 3656 [1982]) beschriebene Methode anwendeten. Die auf 1:1500 mit TRIS-Puffer pH 7,4 (50 mM TRIS-HCl, 150 mM NaCl, 1,0 mM $CaCl_2$ und 0,001 Gew.-%, bezogen auf TRIS-HCl, NaCl und $CaCl_2$ in Lösung, einer neutralen oberflächenaktiven Substanz, wie z.B. Genapol(R)) verdünnte Membransuspension wurde in 5 ml-Portionen mit $^3$H-Nitrendipin (0,1 nM im Test, spez. Aktivität 81,3 Ci/m Mol) und mit den Prüfsubstanzen in verschiedenen Konzentrationen 60 Min. bei 25°C im Schüttelwasserbad inkubiert. Die Abtrennung der Membranfraktionen wurde durch Vakuumfiltration über Whatman-GF/F-Glasfaserfilter vorgenommen und die Radioaktivität im Flüssigkeitsszintillationszähler gemessen. Die unspezifische $^3$H-Nitrendipin-Bindung bestimmten wir in Gegenwart von 1µM Nifedipin. Als charakteristische Größe

wird der $IC_{50}$-Wert bestimmt, d.h. diejenige Konzentration der Testsubstanz, die 50% des radioaktiv markierten Nitrendipins zu verdrängen vermag.

In diesem Modell weisen die erfindungsgemäßen Verbindungen der Formel I $IC_{50}$-Werte von etwa $10^{-6}$ molar bis etwa $10^{-[illegible]}$ molar auf. Sie sind somit deutlich stärker wirksam als bekannte Vergleichsverbindungen wie Flunarizin und Lidoflazin.

Die folgende Tabelle enthält einige der gemessenen $IC_{50}$-Werte.

| Beispiel Nr. | $IC_{50}$ ($10^{-9}$M) | Beispiel Nr. | $IC_{50}$ ($10^{-9}$M) |
|---|---|---|---|
| 16 | 22 | 61 | 60 |
| 17 | 12 | 64 | 19 |
| 18 | 48 | 66 | 12 |
| 22 | 85 | 68 | 0,9 |
| 28 | 65 | 69 | 2 |
| 32 | 12 | 71 | 4,2 |
| 36 | 90 | 73 | 1,2 |
| 37 | 400 | 74 | 1,0 |
| 41 | 8,5 | 75 | 9 |
| 45 | 11 | 81 | 3,6 |
| 50 | 4 | 83 | 70 |
| 56 | 180 | Flunarizin | 1000 |
| | | Lidoflazin | 430 |

Die antianginöse Wirksamkeit der erfindungsgemäßen Verbindungen kann in dem pharmakologischen Testmodell der Coronardurchströmung am isolierten Meerschweinchenherzen gezeigt werden. Für diese Prüfung wurden weibliche heparanisierte (2,5 mg Heparin-Natrium intraperitoneal 1 Std. vor der Tötung) Meerschweinchen (220-250 g) durch einen Schlag auf den Nacken getötet. Unmittelbar nach Öffnung des Thorax wurde durch Kühlung des Herzens mit eiskalter Kochsalzlösung ein Herzstillstand induziert. Die aufsteigende Aorta wurde katheterisiert und entsprechend der Methode von Langendorff an ein nicht-rezirkulierendes Perfusionssystem (hydrostatischer Druck 65 cm $H_2O$) angeschlossen. Nachdem das Herz freigelegt wurde, folgte eine Perfusionsperiode

von 30 Minuten zur Adaptation des Herzens an den isolierten Zustand. Als Perfusat diente eine modifizierte Krebs-Henseleit-Bicarbonatlösung (113.8 mM NaCl, 22.0 mM $NaHCO_3$, 4.7 mM KCl, 1.2 mM $KH_2PO_4$, 1.1 mM $MgSO_4$ x 7 $H_2O$, 2.5 mM $CaCl_2$ x 2 $H_2O$, äquilibriert mit 95% $O_2$ und 5% $CO_2$, pH 7.41, 38°C). Glucose (11 mM) und Natriumpyruvat (2.0 mM) wurden hinzugefügt. Die Osmolalität des Perfusats wurde durch Einstellen der Menge an NaCl konstant gehalten. Die Herzen wurden mit einer Ruhespannung von 2 pond (p) belastet. Herzfrequenz und Kontraktionskraft wurden isometrisch registriert, die Geräte (Fa. Hellige GmbH, Freiburg, Deutschland) dazu auf 2 p geeicht. Der Koronarfluß wurde mit Hilfe eines Tropfenzählers bestimmt, 7 Tropfen entsprechen 1 ml. Die angegebenen Werte sind ausgedrückt als prozentuale Zunahme der Koronardurchströmung nach Applikation der Testsubstanz in der angegebenen Dosierung über einen Zuführungsschlauch (0,1%ige Lösung in Propandiol), bezogen auf Kontrollherzen ohne Zugabe der Testsubstanz. Die folgende Tabelle enthält einige der gemessenen Werte:

| Beispiel Nr. | Dosis (μg) | Zunahme der Koronardurchströmung in % [b] |
|---|---|---|
| 17 | 0.1 | 14-17 |
| | 0.5 | 33-38 |
| 42 | 0.5 | 20-29 |
| 45 | 0.1 | 13-26 |
| | 0.5 | 38-57 |
| 46 | 0.1 | 12-23 |
| | 0.5 | 21-36 |
| 50 | 0.1 | 38 [a] |
| 56 | 1.0 | 40-65 |
| 62 | 0.1 | 9-13 |
| 67 | 0.1 | 5-21 |
| | 0.5 | 23-35 |
| 68 | 0.1 | 20-29 |
| 70 | 0.5 | 36-52 |

[a] Mittelwert aus 28 Messungen

[b] Die angegebenen Werte resultieren aus jeweils zwei Messungen.

In weiteren Testmodellen, mit denen calciumantagonistische Wirkung nachgewiesen werden kann, z. B. an der relaxierenden Wirkung auf das vorkontrahierte Meerschweinchenileum oder am Aktionspotential des isolierten Meerschweinchenkapillarmuskels, sind die Verbindungen der Formel I ebenfalls stark wirksam.

Die erfindungsgemäßen Verbindungen der Formel I und ihre pharmakologisch verträglichen Salze sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Patienten. Bei oraler Dosierung werden befriedigende Ergebnisse mit Dosen von ab 0,01 mg, vorzugsweise ab 0,1 mg und bis zu 100 mg, vorzugsweise bis zu 20 mg einer Verbindung der Formel I pro kg Körpergewicht erreicht. Beim Menschen variiert die tägliche Dosis zwischen 1 und 800 mg, vorzugsweise 2 bis 500 mg, wobei Einzeldosen von 0,5 bis 200 mg, vorzugsweise ein- bis dreimal täglich, gegeben werden können. Für intravenöse und intramuskuläre Anwendung beträgt die Dosis 0,1 bis 300 mg, vorzugsweise 0,5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z. B. Lactose, Dextrose, Rohrzukker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose

und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-, Granulier-, und Dragierverfahren, hergestellt und enthalten 0,1% bis etwa 75%, bevorzugt etwa 1% bis etwa 50% des Wirkstoffs.

Die im Anschluß folgenden Beispiele sollen die Erfindung erläutern, ohne sie auf diese Beispiele zu begrenzen.

A. Herstellung von Zwischenprodukten

Beispiel 1: 5-(2-Chlorethoxy)isochinolin-hydrochlorid

Zu 9,7 g einer 55-60%igen Natriumhydrid-Dispersion in 50 ml absol. Dimethylformamid wurde eine Lösung von 29,2 g (0,2 mol) 5-Hydroxyisochinolin in 300 ml absol. Dimethylformamid so zugetropft, daß die Temperatur auf 45-50°C anstieg, anschließend wurde 2 Std. bei Zimmertemperatur nachgerührt. Die entstandene Anionenlösung wurde nun zu einer Lösung von 49,5 g (0,21 mol) Chlorethyl-p-toluolsulfonat in 100 ml absol. Dimethylformamid zugetropft und 18 Stunden lang nachgerührt.

Das Lösungsmittel wurde abgedampft und der Rückstand zwischen Wasser und Essigester verteilt. Die organische Phase wurde mit 2 N HCl extrahiert, der Extrakt mit konz. NaOH-Lösung auf pH 10 eingestellt und mehrfach mit Methylenchlorid ausgeschüttelt. Diese Extrakte wurden getrocknet und eingeengt. Ausbeute: 34.8 g gelbes Öl.

Zur Herstellung des Hydrochlorids wurde das Rohprodukt in Isopropanol gelöst, mit etherischer Salzsäure sauer gestellt und auf 0°C abgekühlt. Der ausgefallene Niederschlag wurde abgesaugt und mit wenig kaltem Isopropanol und Ether gewaschen und getrocknet. Ausbeute: 33.6 g; Schmp. 191-194°C.

Beispiel 2: 5-(3-Chlorpropoxy )isochinolin-hydrochlorid

Zu 6,6 g einer 55-60%igen Natriumhydrid-Dispersion in 30 ml absol. Dimethylformamid wurde eine Lösung von 20 g (0.138 mol) 5-Hydroxyisochinolin in 200 ml absol. Dimethylformamid so zugetropft, daß die Temperatur etwa 40°C betrug, anschließend 1 Std. bei Zimmertemperatur nachgerührt. Die entstandene Anionenlösung wurde nun zu 65.2 g (0.414 mol) 1-Brom-3-chlorpropan zugetropft und 1 Std. bei 0°C sowie 1 Std. bei Zimmertemperatur gerührt.

Das Lösungsmittel und überschüssiges Bromchlorpropan wurden im Hochvakuum abgezogen und der Rückstand zwischen 2 N NaOH und Methylenchlorid verteilt. Die organische Phase wurde noch zweimal mit Wasser gewaschen, getrocknet und eingeengt.

Die Darstellung des Hydrochlorids erfolgte wie unter Beispiel 1 beschrieben. Ausbeute: 28.1 g; Schmp. 186 - 187°C.

Nach einer analogen Vorgehensweise wurde ebenfalls hergestellt:

5-(4-Chlorbutoxy)-isochinolin-hydrochlorid,
Schmp. 164-167°C.

Beispiel 3: 1-[4,4-Bis(4-methoxyphenyl)butyl]piperazin

a) Bis(4-methoxyphenyl)cyclopropylcarbinol:

Aus 12,7 g (0.53 mol) Magnesiumspänen und 93,5 g (0.50 mol) 4-Bromanisol wurde in 160 ml absol. Ether das Grignard-Reagens hergestellt. Zu dieser Lösung tropfte man langsam 26,0 g (0.228 mol) Cyclopropancarbonsäureethylester und erhitzte anschließend 2,5 Std. zum Rückfluß. Die Reaktionslösung wurde auf Eis gegeben und mit ca. 300 ml gesättigter Ammoniumchloridlösung verdünnt, so daß sich der entstandene Niederschlag löste. Dann wurde mit Ether extrahiert, die etherische Phase mit Wasser gewaschen, getrocknet und eingeengt. Vakuumdestillation des Rohproduktes ergab 39,2 g einer Fraktion bei 195-200°C/0,18 mm Hg.

b) 4-Brom-1,1-bis(4-methoxyphenyl)buten:

39,2 g (0.138 mol) Bis(4-methoxyphenyl)cyclopropylcarbinol wurden mit 165 ml 48% HBr-Lösung versetzt und 4,5 Std. bei 80°C gerührt. Nach dem Abkühlen wurde mit Ether versetzt, die organische Phase abgetrennt, mit gesättigter $NaHCO_3$-Lösung und mit Wasser gewaschen, getrocknet und eingeengt. Ausbeute: 44,0 g eines gelben Öls.
NMR ($CDCl_3$, 60 MHz): δ= 2.66 (t, 2H), 3.33 (t, 2H), 3.72 (s, 3H), 3.78 (s, 3H), 8.34 (t, 1H), 6.6 - 7.2 (m, 8H) ppm.

c) 4-Brom-1,1-bis(4-methoxyphenyl)butan

44 g (0.127 mol) 4-Brom-1,1-bis(4-methoxyphenyl)buten wurden in 160 ml Ethanol gelöst, 2,8 g Palladium/Kohle (10%) zugesetzt und bei Zimmertemperatur innerhalb von etwa 2 Std. in einer Schüttelente hydriert. Dann wurde vom Katalysator abgesaugt, eingeengt und das Rohprodukt durch fraktionierte Destillation gereinigt. Man erhielt 34,4 g einer Fraktion bei 220-226°C/0,2 mm.

d) 1-[4,4-Bis(4-methoxyphenyl)butyl]piperazin

14,0 g (0.04 mol) 4-Brom-1,1-bis(4-methoxyphenyl)butan, 8,2 g (0,052 mol) Piperazin-1-carbonsäureethylester, 11,04 g (0.08 mol) gepulvertes Kaliumcarbonat und 0,6 g Kaliumjodid wurden in 160 ml Methylisobutylketon 30 Std. am Rückfluß gekocht. Nach dem Abkühlen wurde vom Niederschlag abgesaugt und das Lösungsmittel abgedampft.

Das so erhaltene Material wurde in 440 ml Ethanol gelöst,

mit 440 ml 4 N KOH versetzt und 26 Std. am Rückfluß gekocht. Das Ethanol wurde abgedampft, die verbleibende wäßrige Phase mit Methylenchlorid extrahiert, die Extrakte mit 2 N HCl ausgeschüttelt, die saure Phase mehrfach mit Essigester gewaschen, dann mit konz. NaOH auf pH 10 gestellt und mit Methylenchlorid extrahiert. Die Extrakte wurden getrocknet und eingeengt.

Ausbeute: 15,8 g

NMR ($CDCl_3$, 60 MHz): δ= 1,2-3.0 (m, 15H), 3,75 (s, 6H), 3,79 (t, 1H), 6,6-7,2 (m, 8H) ppm.

Nach einer analogen Vorgehensweise wurden ebenfalls hergestellt:

1-[4,4-Bis(4-trifluormethylphenyl)butyl]piperazin,
NMR ($CDCl_3$, 60 MHz): δ= 1.2-3.0 (m, 15H), 3,98 (t, 1H), 7,1-7,6 (m, 8H) ppm;

1-(4,4-Diphenylbutyl)piperazin, Schmp. 58-60°C,
NMR ($CDCl_3$, 60 MHz): δ= 1,2-3,0 (m, 15H), 3,85 (t, 1H), 7,48 (s, 10H) ppm;

1-[4,4-Bis(3-fluorphenyl)butyl]piperazin,
NMR ($CDCl_3$, 60 MHz): δ= 1.2-3.0 (m, 14H), 3,85 (t, 1H), 4,60 (s, 1H), 6,6-7,4 (m, 8H) ppm;

1-[4,4-Bis(4-chlorphenyl)butyl]piperazin, Schmp. 28-32°C;
NMR ($CDCl_3$, 60 MHz): δ= 1,1-3,0 (m, 15H), 3,80 (t, 1H), 6,9-7,3 (m, 8H) ppm.

1-[4,4-Bis(4-fluorphenyl)butyl]piperazin; Schmp. 77-79°C;
NMR ($CDCl_3$, 60 MHz), 1,2-3,0 (m, 15H), 3,83 (t, 1H), 6,6-7,3 (m, 8H) ppm.

<u>Beispiel 4: 1-[3,3-Bis(4-fluorphenyl)propyl]piperazin</u>

a) 2-Cyan-3-(4-fluorphenyl)acrylsäureethylester

117,8 g (0.949 mol) 4-Fluorbenzaldehyd, 118,1 g (1.04 mol) Cyanessigsäureethylester und 2 ml Piperidin wurden in 500

ml Toluol gelöst, langsam zum Rückfluß erhitzt und das Reaktionswasser am Wasserabscheider entfernt. Nach 30 Min. wurde auf etwa das halbe Volumen einggeengt, kaltgestellt und der entstandene Niederschlag abgesaugt. Ausbeute: 171,5 g, Schmp. 96-98°C.

b) 2-Cyan-3,3-bis(4-fluorphenyl)propionsäureethylester

Aus 6,82 g (0.281 mol) Magnesiumspänen und 48,1 g (0.275 mol) 4-Bromfluorbenzol wurde in 150 ml absol. Ether ein Grignard-Reagens hergestellt. Zu dieser Lösung tropfte man während 10-15 Minuten eine Lösung von 54,8 g (0.250 mol) 2-Cyan-3-(4-fluorphenyl)acrylsäureethylester in 200 ml Toluol so zu, daß die Temperatur auf ca. 90°C anstieg und der Ether abdestillierte. Danach wurde noch 30 Min. zum Rückfluß erhitzt, nach dem Abkühlen auf 300 ml Eis und 15 ml konz. $H_2SO_4$ gegeben, die wäßrige Phase noch einmal mit Toluol extrahiert, die vereinigten organischen Phasen mit Wasser gewaschen, getrocknet und eingeengt. Ausbeute: 79,1 g gelbe Kristalle. Schmp. 99-101°C (aus Isopropanol/n-Hexan).

c) 3,3-Bis(4-fluorphenyl)propionsäure

76,2 g (0.242 mol) 2-Cyan-3,3-bis(4-fluorphenyl)propionsäureethylester wurden heiß in 250 ml Eisessig gelöst, 250 ml halbkonz. $H_2SO_4$ zugegeben und 18 Std. zum Rückfluß erhitzt. Danach wurde auf 1 kg Eis gegeben, mehrfach mit Ether extrahiert, die Extrakte mit Wasser gewaschen, getrocknet und eingeengt. Ausbeute: 60,3 g gelbes Öl, das allmählich kristallisierte. Schmp. 97-99°C (aus Cyclohexan).

d) 3,3-Bis(4-fluorphenyl)propanol

57,9 g (0.221 mol) 3,3-Bis(4-fluorphenyl)propionsäure wurden in 200 ml absol. Ether gelöst und zu einer Suspension von 10,9 g (0.287 mol) $LiAlH_4$ in 150 ml absol. Ether getropft. Nach Beendigung des Zutropfens wurde noch 30 Min. nachgerührt und zur Hydrolyse nacheinander 175 ml gesättigte Seignettesalzlösung und 400 ml 10% $H_2SO_4$ zugetropft.

Nach Filtration wurden die Phasen getrennt, die etherische Phase mit $NaHCO_3$- und NaCl-Lösung gewaschen, getrocknet und eingeengt.
Ausbeute: 50,7 g gelbes Öl.
NMR ($CDCl_3$, 60 MHz): δ= 2,0-2,4 (m, 3H), 3,48 (t, 2H), 4,01 (t, 1H), 6,6-7,2 (m, 8H) ppm;

e) 3,3-Bis(4-fluorphenyl)propylbromid

50,1 g (0.202 mol) 3,3-Bis(4-fluorphenyl)propanol wurden in 150 ml Toluol gelöst und eine Lösung von 21,8 g (0.081 mol) $PBr_3$ in 50 ml Toluol zugetropft. Es wurde 30 Minuten bei Zimmertemperatur und 90 Minuten bei 60°C gerührt, zur Hydrolyse auf 400 ml Eis gegeben, 200 ml Ether zugesetzt, die organische Phase abgetrennt, mit $NaHCO_3$- und NaCl-Lösung gewaschen, getrocknet, eingeengt und zur Reinigung im Vakuum destilliert. Ausbeute: 32,2 farbloses Öl, Siedep. 144-162°C/0,6 mm.

f) 1-[3,3-Bis(4-fluorphenyl)propyl]piperazin

31,1 g (0.100 mol) 3,3-Bis(4-fluorphenyl)propylbromid, 20,6 g (0.130 mol) Piperazin-1-carbonsäureethylester, 27,6 g (0.200 mol) gepulvertes Kaliumcarbonat und 4,15 g Kaliumjodid wurden in 400 ml Toluol 31 Stunden zum Rückfluß erhitzt; dann wurde vom Niederschlag abfiltriert und eingeengt. Das so erhaltene Material wurde in 450 ml Ethanol gelöst, mit 450 ml 4 N KOH versetzt und 9 Std. zum Rückfluß erhitzt. Nach der üblichen Aufarbeitung (vgl. Beispiel 3d) erhielt man 26,8 g Produkt, Schmp. 58-64°C.

Beispiel 5 : 1-[2,2-Bis(4-fluorphenyl)ethyl]piperazin

a) Bis(4-fluorphenyl)acetylchlorid

35,5 g (0.143 mol) Bis(4-fluorphenyl)essigsäure wurden in 150 ml Thionylchlorid 4,5 Stunden am Rückfluß gerührt und das überschüssige Thionylchlorid im Vakuum abgedampft.
Ausbeute: 38,1 g

b) Bis(4-fluorphenyl)essigsäure-N-benzylpiperazid

25,2 g (0.143 mol) N-Benzylpiperazin und 28,9 g (0.286 mol) Triethylamin wurden in 150 ml Toluol vorgelegt und bei 0-5°C innerhalb 1 Stunde eine Lösung von 38,1 g (0.143 mol) Bis(4-fluorphenyl)acetylchlorid in 100 ml Toluol zugetropft. Nach 2 Stunden Rühren bei Zimmertemperatur wurde vom ausgefallenen Niederschlag abgesaugt und das Filtrat eingeengt. Ausbeute: 58 g.
NMR ($CDCl_3$, 60 MHz), δ= 2,0-2,7 (m, 4H), 3,2-3,9 (m, 6H), 5,12 (s, 1H), 6,7-7,4 (m, 13H) ppm.

c) 1-Benzyl-4-[2,2-bis(4-fluorphenyl)ethyl]piperazin

Eine Lösung von 58 g (0.143 mol) Bis(4-fluorphenyl)-essigsäure-N-benzylpiperazid in 270 ml absol. THF wurde langsam zu einer Suspension von 10,8 g (0.286 mol) $LiAlH_4$ in 270 ml absol. THF zugetropft, und danach wurde ca. 2 Stunden zum Rückfluß erhitzt. Zur Hydrolyse wurden 10 ml $H_2O$ zugetropft, dann 50 ml 2 N NaOH und erneut 10 ml $H_2O$. Vom ausgefallenen Niederschlag wurde abgesaugt, mehrmals mit THF gewaschen, getrocknet und eingeengt. Das Rohprodukt wurde über das Hydrochlorid gereinigt. Ausbeute: 41,8 g Hydrochlorid, Schmp. 242-246°C (Zers.) (aus Isopropanol).

d) 1-[2,2-Bis(4-fluorphenyl)ethyl]piperazin

35,1 g 1-Benzyl-4-[2,2-bis(4-fluorphenyl)ethyl]-piperazin-hydrochlorid wurden in 1400 ml Methanol an 8 g Palladium/Kohle (10%) während 1 Stunde bei Zimmertemperatur hydriert. Der Katalysator wurde abgesaugt, das Lösungsmittel abgedampft, der Rückstand in 200 ml Wasser gelöst, mit 2 N NaOH auf pH 10 eingestellt, mehrfach mit Methylenchlorid extrahiert, die Extrakte getrocknet und eingeengt. Ausbeute: 17,8 g
NMR ($CDCl_3$, 60 MHz), δ: 2,03 (s, 1H), 2,3-3,0 (m, 10H), 4,12 (t, 1H), 6,6-7,3 (m, 8H) ppm.

Beispiel 6: 1-[4,5-Bis(4-fluorphenyl)pentyl]piperazin

a) 4,5-Bis(4-fluorphenyl)-4-hydroxypentylchlorid

Aus 13,8 g (0.575 mol) Magnesium-Spänen und 83,1 g

(0.575 mol) 4-Fluorbenzylchlorid in 560 ml absol. Ether wurde eine Grignard-Lösung bereitet. Diese Lösung wurde zu einer Lösung von 92 g (0.46 mol) ω-Chlor-4-fluorbutyrophenon in 300 ml absol. Ether hinzugetropft und 2 Stunden zum Rückfluß erhitzt. Dann wurde auf 500 ml Eis gegeben, mit 1 l $NH_4Cl$-Lösung versetzt, mehrfach mit Ether extrahiert und die organischen Phasen getrocknet und eingeengt. Ausbeute: 143 gelbes Öl.
NMR ($CDCl_3$, 60 MHz), δ: 1,2-2,3 (m, 5H), 3,0-3,6 (m, 4H), 6,6-7,4 (m, 8H) ppm.

b) 4,5-Bis(4-fluorphenyl)pentylchlorid

165 g (0.53 mol) 4,5-Bis(4-fluorphenyl)-4-hydroxypentylchlorid wurden in 1650 ml Eisessig unter Zusatz von 16,5 g Palladium/Kohle (10%) und 80 ml konz. $H_2SO_4$ während 5 Stunden bei Zimmertemperatur hydriert. Es wurde vom Katalysator abgesaugt, auf etwa ein Viertel des Volumens eingeengt, mit Methylenchlorid und Wasser versetzt, mit konz. NaOH auf pH 6 gestellt, die organische Phase abgetrennt, mit $NaHCO_3$-Lösung und Wasser gewaschen, getrocknet und eingeengt. Ausbeute: 129 g
NMR ($CDCl_3$, 60 MHz), δ: 1,3-1,9 (m, 4H), 2,8 (br, s, 2H), 3,39 (t, 2H), 3,92 (t, 1H), 6,6-7,1 (m, 8H) ppm.

c) 1-[4,5-Bis(4-fluorphenyl)pentyl]piperazin

50 g (0.17 mol) 4,5-Bis(4-fluorphenyl)pentylchlorid, 32,5 g (0.21 mol) Piperazin-1-carbonsäureethylester, 46,9 g (0.34 mol) gepulvertes Kaliumcarbonat und 2,8 g Kaliumjodid wurden in 580 ml Methylisobutylketon analog dem in Beispiel 3d) angegebenen Verfahren umgesetzt und aufgearbeitet. Ausbeute: 39,2 g.
NMR ($CDCl_3$, 60 MHz), δ: 1,1-1,8 (m, 4H), 2,0-2,5 (m, 8H), 2,6-3,0 (m, 6H), 6,7-7,0 (m, 8H) ppm.

Beispiel 7: 4-[4,4-Bis(4-fluorphenyl)butyl]piperidin

a) 1-Benzyl-4-[4,4-bis(4-fluorphenyl)butyl]-4-hydroxypiperidin

Aus 3,17 g (0.132 mol) Magnesiumspänen und 42,8 g (0.132 mol) 4,4-Bis(4-fluorphenyl)butylbromid wurde in

85 ml absol. THF eine Grignard-Lösung bereitet. Innerhalb von 30 Min. wurde dann eine Lösung von 25 g (0.132 mol) destilliertem N-Benzylpiperidon in 130 ml absol. THF zugetropft und 1,5 Stunden bei Zimmertemperatur nachgerührt. Das Reaktionsgemisch wurde auf 800 ml Eis gegeben, mit 400 ml gesättigter $NH_4Cl$-Lösung versetzt und mehrfach mit Ether extrahiert. Die Extrakte wurden getrocknet und eingeengt. Ausbeute: 54,6 g.
NMR ($CDCl_3$, 60 MHz), δ: 1,0-3,3 (m, 16H), 3,50 (s, 2H), 3,85 (t, 1H), 6,7-7,3 (m, 13H) ppm.

b) 1-Benzyl-4-[4,4-bis(4-fluorphenyl)butyliden]-piperidin (und Isomeres)

51 g (0.117 mol) rohes 1-Benzyl-4-[4,4-bis(4-fluorphenyl)-butyl]-4-hydroxypiperidin wurden in 400 ml 85% Phosphorsäure 1,5 Stunden bei 140°C und 1 Stunde bei 160°C gerührt. Die Reaktionslösung wurde auf 1 kg Eis gegeben und auf pH 9 gestellt, mit Methylenchlorid extrahiert, die Extrakte mit Wasser gewaschen, getrocknet, eingeengt und das Rohprodukt durch Flash-Säulenchromatographie an 800 g Kieselgel mit $CH_2Cl_2/CH_3OH$ (99:1) gereinigt. Ausbeute: 18,3 g.
MS (70 eV): $M^+ = 417$.

c) 4-[4,4-Bis(4-fluorphenyl)butyl]piperidin

17 g (40.7 mmol) 1-Benzyl-4-[4,4-bis(4-fluorphenyl)-butyliden]piperidin wurden in 850 ml Methanol an 5 g Palladium/Kohle (10%) während 2 Stunden bei 45°C in einer Schüttelente hydriert. Der Katalysator wurde abgesaugt und die Lösung eingeengt. Ausbeute: 10,6 g.
NMR ($CDCl_3$, 60 MHz): δ = 1,0-3,4 (m, 15H), 3,85 (t, 1H), 4,30 (s, 1H), 6,7-7,3 (m, 8H) ppm.

Beispiel 8: 4-N-[4,4-Bis(4-fluorphenyl)butyl]-N-methyl-aminopiperidin

a) 1-Benzyl-4-methylaminopiperidin

Zu einer Suspension von 4,37 g (0.115 mol) $LiAlH_4$ in 90 ml absol. Ether wurden eine Suspension von 20 g (0.076 mol)

1-Benzyl-4-ethoxycarbonylaminopiperidin in 90 ml absol. Ether sowie 50 ml absol. THF zugegeben und 4,5 Stunden zum Rückfluß erhitzt. Unter Kühlung wurden nacheinander 5 ml $H_2O$, 5 ml 2N NaOH und erneut 25 ml $H_2O$ zugetropft. Vom entstandenen Niederschlag wurde abgesaugt, mehrmals mit Ether nachgewaschen, getrocknet und eingeengt.
Ausbeute: 15,3 g.
NMR ($CDCl_3$, 60 MHz), δ= 1,0-3,0 (m, 10H), 2,38 (s, 3H), 3,46 (s, 2H), 7,20 (s, 5H) ppm.

b) 1-Benzyl-4-N-[4,4-Bis(4-fluorphenyl)butyl]-N-methylaminopiperidin

15,3 g (75 mmol) 1-Benzyl-4-methylaminopiperidin, 24,4 g (75 mmol) 4,4-Bis(4-fluorphenyl)butylbromid, 20,7 g (150 mmol) gepulvertes Kaliumcarbonat und 1,2 g Kaliumjodid wurden in 200 ml Methylisobutylketon 63 Stunden am Rückfluß gekocht. Vom Niederschlag wurde abgesaugt, eingeengt, und das Rohprodukt durch Flash-Säulenchromatographie an Kieselgel mit $CH_2Cl_2/CH_3OH$ (99:1 bis 9:1) gereinigt.
Ausbeute: 25,6 g
NMR ($CDCl_3$, 60 MHz), δ = 1,2-3,1 (m, 15H), 2,12 (s, 3H), 3,41 (s, 2H), 3,80 (t, 1H), 6,7-7,3 (m, 13H) ppm.

c) 4-N[4,4-Bis(4-fluorphenyl)butyl]-N-methylaminopiperidin

17,7 g (40 mmol) 1-Benzyl-4-N-[4,4-bis-(4-fluorphenyl)-butyl]-N-methylaminopiperidin wurden in 900 ml 4,4% methanolischer Ameisensäure gelöst, unter Schutzgas zu einer Suspension von 17,7 g Palladium/Kohle (10%) in 900 ml 4,4% methanolischer Ameisensäure zugetropft und 90 Min. zum Rückfluß erhitzt. Vom Katalysator wurde abgesaugt, die Reaktionslösung zur Trockne eingeengt, in wenig Wasser aufgenommen, mit 2 N NaOH auf pH 10 gestellt und mit Methylenchlorid extrahiert. Die Extrakte wurden getrocknet und eingeengt. Ausbeute: 10,9 g
NMR ($CDCl_3$, 60 MHz): δ = 1,2-3,4 (m, 16H), 2,20 (s, 3H) 3,83 (t, 1H), 6,7-7,3 (m, 8H) ppm.

Beispiel 9: 1-[4,4-Bis(4-fluorphenyl)butyl]homopiperazin

24 g (0.24 mol) 1,4-Diazacycloheptan, 7,8 g (0.024 mol) 4,4-Bis(4-fluorphenyl)butylbromid und 0,14 g Kaliumjodid wurden in 60 ml Dimethylformamid 4 Std. bei Zimmertemperatur gerührt. Das Lösungsmittel wurde im Vakuum abgezogen, der Rückstand in Methylenchlorid aufgenommen, mit 2 N HCl ausgeschüttelt, die saure Phase mit konz. NaOH auf pH 11 gestellt, mit Methylenchlorid extrahiert, die Extrakte getrocknet und eingeengt. Ausbeute: 7,15 g.
NMR ($CDCl_3$, 60 MHz): δ 1,2-3,2 (m, 16H), 3,81 (t, 1H), 4,50 (s, 1H), 6,7-7,3 (m, 8H) ppm.

Beispiel 10: 1-[4,4-Bis(4-fluorphenyl)butyl]-4-methylaminopiperidin

a) 4-Ethoxycarbonylaminopiperidin

16 g (61 mmol) 1-Benzyl-4-ethoxycarbonylaminopiperidin wurden in 240 ml Methanol und 80 ml 10% methanolischer Salzsäure an 4 g Palladium/Kohle (10%) bei 45°C während 90 Min. in einer Schüttelente hydriert. Der Katalysator wurde abgesaugt, das Lösungsmittel abgedampft, der Rückstand in 400 ml Ethanol gelöst und mit ethanolischer NaOH neutralisiert. Vom ausgefallenen NaCl wurde abfiltriert und eingeengt. Ausbeute: 10,5 g; Schmp. 192 - 194°C.

b) 1-[4,4-Bis(4-fluorphenyl)butyl]-4-ethoxycarbonylaminopiperidin

11 g (64 mmol) 4-Ethoxycarbonylaminopiperidin, 20,5 g (64 mmol) 4,4-Bis(4-fluorphenyl)butylbromid, 12,9 g (128 mmol) Triethylamin und 0,18 g Kaliumjodid wurden in 180 ml absol. Dimethylformamid 36 Std. bei 80°C gerührt, mit ca. 1,5 l Wasser verdünnt, 100 ml 2N NaOH zugesetzt und zweimal mit Methylenchlorid extrahiert. Die organischen Phasen wurden getrocknet, eingeengt und das Rohprodukt durch Flashchromatographie an Kieselgel mit $CH_2Cl_2/CH_3OH$ (95:5) gereinigt. Das so erhaltene Produkt (22,9 g) wurde in 350 ml Cyclohexan heiß gelöst. Es wurde von unlöslichem Rückstand abdekantiert, auf die Hälfte eingeengt, gekühlt

und der Niederschlag abgesaugt.
Ausbeute: 13,0 g; Schmp. 115 - 116°C.

c) 1-[4,4-Bis(4-fluorphenyl)butyl]-4-methylaminopiperidin

13,0 g (31.3 mmol) 1-[4,4-Bis(4-fluorphenyl)butyl]-4-ethoxycarbonylaminopiperidin wurden in 60 ml absol. THF gelöst und zu einer Suspension von 1,82 g (46.9 mmol) $LiAlH_4$ in 60 ml absol. THF zugetropft, 1 Stunde bei Zimmertemperatur und 1 Stunde bei 40°C gerührt. Unter Kühlung wurden nacheinander 1,8 ml $H_2O$, 1,8 ml 2N NaOH und erneut 9 ml $H_2O$ zugetropft; dann wurde vom entstandenen Niederschlag abgesaugt, mit wenig THF nachgewaschen, getrocknet und eingeengt.
Ausbeute: 9,15 g.
NMR ($CDCl_3$, 60 MHz): δ=1,0-3,0 (m, 16H), 2,42 (s, 3H), 3,86 (t, 1H), 6,7-7,3 (m, 8H) ppm.

Beispiel 11: 1-[4,4-Bis(4-fluorphenyl)butyl]piperazin-2-carboxamid

a) 4-Tritylpiperazin-2-carboxamid

1,29 g (10 mmol) Piperazin-2-carboxamid wurden in 20 ml absolutem Methylenchlorid gelöst, 1,01 g (10 mmol) Triethylamin zugegeben, tropfenweise eine Lösung von 2,79 g (10 mmol) Triphenylmethylchlorid in 20 ml absol. Methylenchlorid hinzugefügt und 5 Stunden bei Zimmertemperatur nachgerührt. Die Reaktionslösung wurde zweimal mit Wasser gewaschen, getrocknet, eingeengt und das Rohprodukt durch Flash-Chromatographie an Kieselgel mit $CH_2Cl_2/CH_3OH$ 95:5 gereinigt. Ausbeute: 2,5 g; Schmp. 229-230°C.

b) 1-[4,4-Bis(4-fluorphenyl)butyl]-4-tritylpiperazin-2-carboxamid

20 g (53.9 mmol) 4-Tritylpiperazin-2-carboxamid, 17,5 g (53.9 mmol) 4,4-Bis(4-fluorphenyl)butylbromid, 14,9 g (108 mmol) gepulvertes Kaliumcarbonat und 0,5 g Kaliumjodid wurden in 270 ml Methylisobutylketon 72 Stunden am Rückfluß

gekocht. Es wurde vom Niederschlag abfiltriert und das Lösungsmittel im Vakuum abgezogen.
Ausbeute: 33,1 g; Schmp. 190-191°C (aus Aceton).

c) 1-[4,4-Bis(4-fluorphenyl)butyl]-piperazin-2-carboxamid

33,1 g (53.8 mmol) 1-[4,4-Bis(4-fluorphenyl)butyl]-4-trityl-piperazin-2-carboxamid wurden in 180 ml Eisessig gelöst, 180 ml Wasser innerhalb von 15 Min. zugetropft, 90 Min. nachgerührt, vom öligen Niederschlag abdekantiert, 720 ml $H_2O$ und etwas Celite$^{(R)}$, einem Produkt der Firma Johns-Mannville Corporation, New York, zugesetzt, filtriert, mit konz. NaOH auf pH 10 gestellt und mit Methylenchlorid extrahiert. Die organische Phase wurde getrocknet und im Vakuum eingeengt. Ausbeute: 12,2 g
NMR ($CDCl_3$, 270 MHz): 1,3-1,6 (m, 2H), 1,85-2,4 (m, 4H), 2,5-3,0 (m, 9H), 3,1-3,25 (m, 1H), 3,85 (t, 1H), 5,6-5,9 (m, 1H), 6,8-7,2 (m, 9H) ppm.

Beispiel 12: 4-[4,4-Bis(4-fluorphenyl)butyl]piperazin-2-carbonsäure

37,5 g (90.8 mmol) 7-[4,4-Bis(4-fluorphenyl)butyl]-hexahydro-3,3-dimethylimidazo[1,5-a]pyrazin-1(5H)-on wurden in 375 ml 1N HCl 2,5 Std. zum Rückfluß erhitzt. Nach dem Abkühlen wurde mit Methylenchlorid gewaschen, die wäßrige Phase mit konz. NaOH auf pH 9 gestellt, zweimal mit Methylenchlorid extrahiert, die Extrakte getrocknet und eingeengt.
Ausbeute: 30,9 g; Schmp. 184-187°C.

Beispiel 13: 1-[4,4-Bis(4-fluorphenyl)butyl]-4-(2-chlorethyl)-piperidin

a) 1-[4,4-Bis(4-fluorphenyl)butyl]-4-(2-hydroxyethyl)-piperidin

20 g (62 mmol) 4,4-Bis(4-fluorphenyl)butylbromid, 8,8 g (68 mmol) 4-Hydroxyethylpiperidin, 12,5 g (124 mmol) Triethylamin und 0,1 g Kaliumjodid wurden in 200 ml absol. Dimethylformamid 4,5 Stunden bei 80°C gerührt. Das Lösungsmittel wurde im Vakuum abgezogen, der Rückstand in Methylenchlorid aufgenommen, mit 2 N HCl, Wasser und NaCl-Lösung ausgeschüttelt, getrocknet und eingeengt. Ausbeute: 23 g. NMR ($CDCl_3$, 60 MHz), δ = 1,2-4,0 (m, 21H), 6,7-7,2 (m, 8H) ppm.

b) 1-[4,4-Bis(4-fluorphenyl)butyl]-4-(2-chlorethyl)piperidin

23 g (61.6 mmol) 1-[4,4-Bis(4-fluorphenyl)butyl]-4-(2-hydroxyethyl)piperidin wurden in 100 ml Methylenchlorid unter Eiskühlung mit 115 g Thionylchlorid tropfenweise versetzt und anschließend 5,5 Stunden zum Rückfluß erhitzt. Lösungsmittel und überschüssiges Reagens wurden im Vakuum abgezogen, der Rückstand (das Hydrochlorid des Produktes) durch Zugabe von 450 ml Ether kristallisiert, abgesaugt, mehrere Male mit Ether gewaschen und im Vakuum getrocknet. Ausbeute: 22,8 g, Schmp. 110-112°C.

B. Herstellung von Endprodukten der Formel I

Beispiel 14: 5-[2-[4-[4,4-Bis(4-methoxyphenyl)butyl]-piperazin-1-yl]-ethoxy]isochinolin

3,1 g (15 mmol) 5-(2-Chlorethoxy)isochinolin, 5,27 g (15 mmol) 1-[4,4-Bis(4-methoxyphenyl)butyl]piperazin, 3,0 g (30 mmol) Triethylamin und 50 mg Kaliumjodid wurden in 30 ml Dimethylformamid 26 Stunden bei 80°C gerührt. Die Reaktionslösung wurde mit 250 ml Wasser verdünnt, zweimal mit Methylenchlorid extrahiert, die Extrakte im Vakuum eingedampft und der Rückstand durch Säulenchromatographie an Kieselgel mit $CH_2Cl_2/CH_3OH$ (99:1 bis 95:5) gereinigt. Ausbeute: 3,7 g.

Zur Herstellung des Dimaleinats wurden 3,7 g der Base in 100 ml Isopropanol gelöst, eine Lösung von 1,64 g Maleinsäure in 20 ml Isopropanol zugesetzt und der ausgefallene Niederschlag abgesaugt und getrocknet. Ausbeute: 4,6 g; Schmp. 159-161°C (Zers.)

Beispiel 15: 6-[3-[4-[4,4-Bis(4-fluorphenyl)butyl]piperazin-1-yl]-propoxy]-1,2,3,4-tetrahydrochinolin-2-on

2,84 g (10 mmol) 6-(3-Brompropoxy)-1,2,3,4-tetrahydrochinolin-2-on, 3,30 g (10 mmol) 1-[4,4-Bis(4-fluorphenyl)-butyl]piperazin, 4,15 g gepulvertes Kaliumcarbonat und 0,42 g Kaliumjodid wurden in 40 ml Butanon 6 Stunden am Rückfluß gekocht. Vom Niederschlag wurde abgesaugt, das Filtrat eingeengt, in Methylenchlorid aufgenommen, mit 2 N NaOH gewaschen, getrocknet, eingeengt und das Rohprodukt durch Säulenchromatographie an Kieselgel mit $CH_2Cl_2/CH_3OH$ 94:6 gereinigt.
Ausbeute: 3,95 g.

Zur Herstellung des Dicitrats wurden 3,80 g der Base in 50 ml Isopropanol gelöst und in der Wärme eine Lösung von 2,74 g Citronensäure in 50 ml Isopropanol zugegeben. Der in der Kälte ausgefallene Niederschlag wurde abgesaugt und aus Isopropanol umkristallisiert.
Ausbeute: 2,95 g, sintert ab ca. 80°C.

Beispiel 16: 1-[4,4-Bis(4-fluorphenyl)butyl]-4-[(2-cyclohexyloxy)-ethyl]piperazin

5,0 g (15.4 mmol) 4,4-Bis(4-fluorphenyl)butylbromid, 3,26 g (15.4 mmol) 1-(2-Cyclohexyloxy-ethyl)piperazin, 3,11 g (30.8 mmol) Triethylamin und 54 mg Kaliumjodid wurden in 35 ml absolutem Dimethylformamid 6 Stunden bei 80°C gerührt. Das Lösungsmittel wurde im Vakuum abgezogen, der Rückstand in Methylenchlorid aufgenommen, mit 2 N NaOH und Wasser gewaschen, getrocknet, eingeengt und durch Säulenchromatographie an Kieselgel mit $CH_2Cl_2/CH_3OH$ (99:1 bis 93:7) gereinigt.
Ausbeute: 4,15 g farbloses Öl.

Zur Herstellung des Dimaleinats wurden 4,00 g der Base in 50 ml Aceton gelöst, bei Zimmertemperatur eine Lösung von 2,04 g Maleinsäure in 20 ml Aceton zugegeben, der entstandene Niederschlag abgesaugt und getrocknet. Ausbeute: 5,40 g; Schmp. 189-192°C.

Beispiel 17: 1-[4,4-Bis(4-fluorphenyl)butyl]-4-[2-(4-methylphenoxy)ethyl]piperazin

Zu 0,5 g Natriumhydrid-Dispersion (55%) in 8 ml absol. Dimethylformamid wurde eine Lösung von 1,18 g (11 mmol) p-Kresol in 20 ml absol. Dimethylformamid langsam zugetropft und 1 Stunde bei 40°C nachgerührt. Bei Zimmertemperatur wurde dann eine Lösung von 4,85 g (12.4 mmol) 1-[4,4-Bis-(4-fluorphenyl)butyl]-4-(2-chlorethyl)piperazin in 20 ml absol. Dimethylformamid zugegeben und 48 Stunden bei Zimmertemperatur gerührt. Das Lösungsmittel wurde im Vakuum abgezogen, der Rückstand in Methylenchlorid gelöst und mit 2 N NaOH und Wasser gewaschen, getrocknet, eingeengt und durch Säulenchromatographie an Kieselgel mit Toluol/Ethanol (99:1 bis 80:20) gereinigt.
Ausbeute: 3,3 g.

Zur Herstellung des Dihydrochlorids wurden 3,2 g der Base in 15 ml Isopropanol gelöst und mit ethanolischer HCl auf pH 3 gestellt. Zur Vervollständigung der Fällung wurden langsam 30 ml Ether zugesetzt, der Niederschlag abgesaugt und getrocknet.
Ausbeute: 3,3 g; Schmp. 203-205°C.

Beispiel 18: 1-[4,4-Bis(4-fluorphenyl)butyl]-4-[2-(1-naphthyloxy)ethyl]piperazin

2,0 g (5,1 mmol) 1-[4,4-Bis(4-fluorphenyl)butyl]-4-(2-chlorethyl)piperazin, 0,73 g (5.1 mmol) α-Naphthol, 1,41 g (10.2 mmol) gemahlenes Kaliumcarbonat und 50 mg Kaliumjodid wurden in 10 ml Butanon 7 Stunden zum Rückfluß erhitzt. Das Lösungsmittel wurde abgedampft, der Rückstand zwischen Wasser und Methylenchlorid verteilt, der pH auf 8-9 eingestellt, die Phasen getrennt, die organische Phase getrocknet, eingeengt und das Rohprodukt durch Säulenchromatographie an Kieselgel mit $CH_2Cl_2$ und $CH_2Cl_2/CH_3OH$ 99:1

gereinigt.
Ausbeute: 1,25 g.

Zur Herstellung des Dimaleinats wurden 1,1 g der Base in 20 ml Isopropanol gelöst und eine Lösung von 0,51 g Maleinsäure in 10 ml Isopropanol zugegeben. Der ausgefallene Niederschlag wurde abgesaugt und getrocknet.
Ausbeute: 1,35 g; Schmp. 187-189°C.

Unter Verwendung geeigneter Ausgangsstoffe und Reagenzien erhielt man die im folgenden tabellarisch aufgeführten Verbindungen unter Anwendung der in den Beispielen 14-18 und 96-97 beschriebenen Verfahrensweisen.

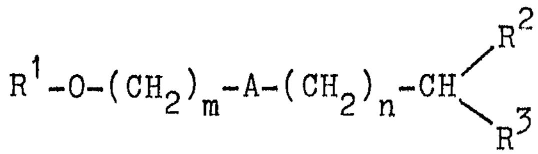

| Bsp. | $R^1$ | $R^2$ | $R^3$ | A | m | n | Salz | Schmp (°C) |
|---|---|---|---|---|---|---|---|---|
| 19 | 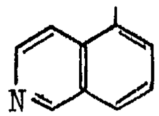 | 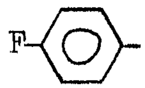 | 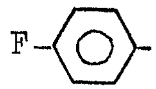 | 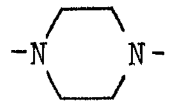 | 2 | 3 | Dimaleinat | 159-161 (Z.) |
| 20 | " | " | " | " | 2 | 3 | Dicitrat | Sintert ab 95 |
| 21 | " | " | " | " | 3 | 3 | Dimaleinat | 164-165 (Z.) |
| 22 | " | " | " | " | 4 | 3 | " | 166-167 (Z.) |
| 23 | " | " | " | " | 2 | 2 | " | 157-159 (Z.) |
| 24 | 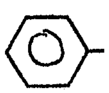 | " | " | " | 2 | 3 | " | 182-185 |

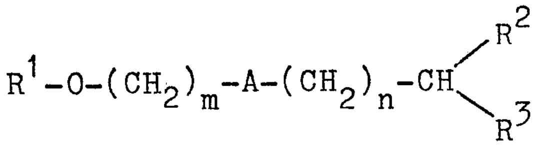

| Bsp. | $R^1$ | $R^2$ | $R^3$ | A | m | n | Salz | Schmp (°C) |
|---|---|---|---|---|---|---|---|---|
| 25 | 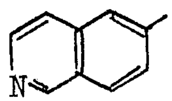 | 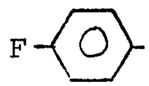 | 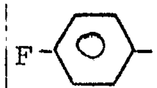 | 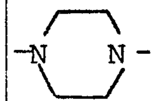 | 2 | 3 | Dicitrat | Sintert ab 110 |
| 26 | 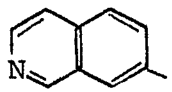 | " | " | " | 2 | 3 | Dimaleinat | 154-157 (Z.) |
| 27 | 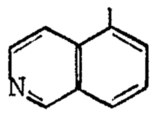 | " | " | 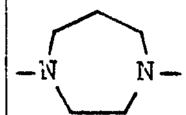 | 2 | 3 | Dimaleinat | 122-126 (Z.) |
| 28 | 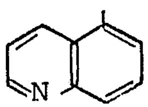 | " | " | 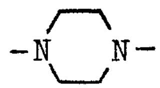 | 2 | 3 | " | 164-167 |
| 29 | 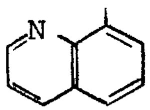 | " | " | " | 2 | 3 | " | 161-163 |
| 30 | 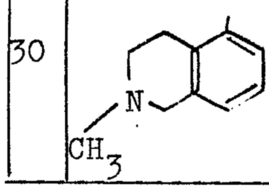 | " | " | " | 2 | 3 | Trimaleinat | 133-135 |

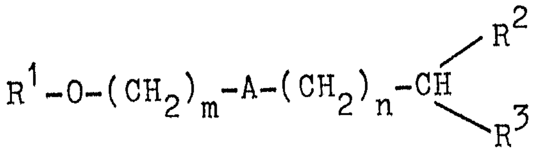

| Bsp. | $R^1$ | $R^2$ | $R^3$ | A | m | n | Salz | Schmp (°C) |
|---|---|---|---|---|---|---|---|---|
| 31 | 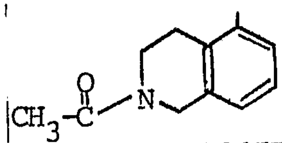 | 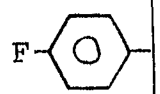 | 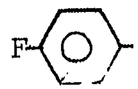 | 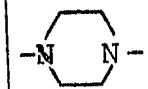 | 2 | 3 | Dimaleinat | 178-180 |
| 32 | 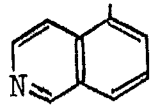 | " | " | 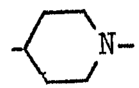 | 2 | 3 | Dioxalat | 100-103 (Z.) |
| 33 | " | 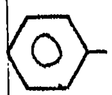 | 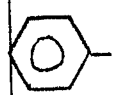 | 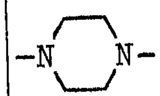 | 2 | 3 | Dimaleinat | 164 (Z.) |
| 34 | " | 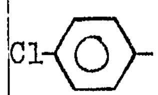 | 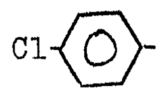 | " | 2 | 3 | " | 165 (Z.) |
| 35 | " | 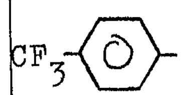 | 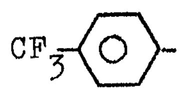 | " | 2 | 3 | Dimaleinat | 164-167 (Z.) |
| 36 | " | 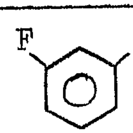 | 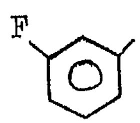 | " | 2 | 3 | " | 158-159 (Z.) |

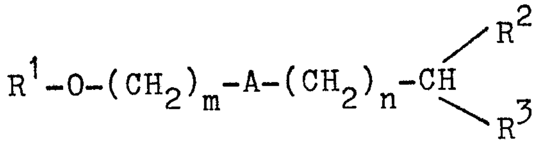

| Bsp. | $R^1$ | $R^2$ | $R^3$ | A | m | n | Salz | Schmp (°C) |
|---|---|---|---|---|---|---|---|---|
| 37 | 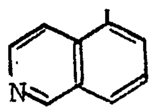 | 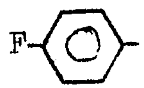 | 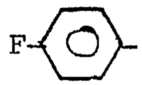 | 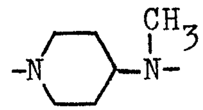 | 2 | 3 | Dimaleinat | 156 (Z.) |
| 38 | " | " | " | 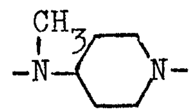 | 2 | 3 | " | 164 |
| 39 | " | " | " | 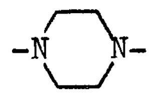 | 2 | 4 | " | 166-167 (Z.) |
| 40 | " | " | " | " | 2 | 1 | " | 165-166 |
| 41 | 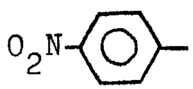 | " | " | " | 2 | 3 | Dihydro-chlorid | 154-157 |
| 42 | 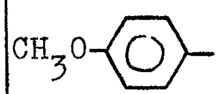 | " | " | " | 2 | 3 | " | 204-207 |

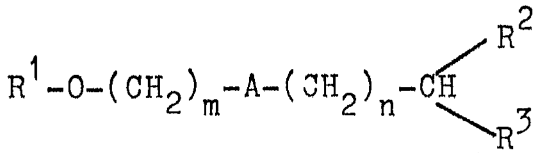

| Bsp. | $R^1$ | $R^2$ | $R^3$ | A | m | n | Salz | Schmp (°C) |
|---|---|---|---|---|---|---|---|---|
| 43 | 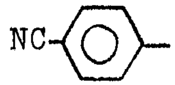 | 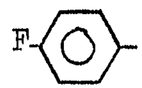 | 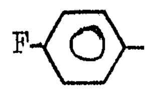 | 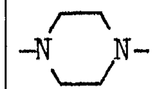 | 2 | 3 | Dihydro-chlorid | 214-218 |
| 44 | 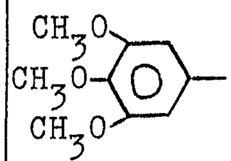 | " | " | " | 2 | 3 | " | 212 (Z.) |
| 45 | 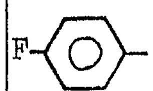 | " | " | " | 2 | 3 | " | 210-212 |
| 46 | 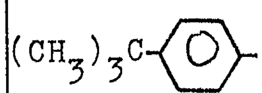 | " | " | " | 2 | 3 | " | 214-217 |
| 47 | 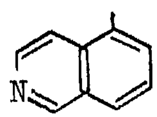 | " | " | 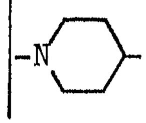 | 2 | 3 | Dioxalat | 105-109 |

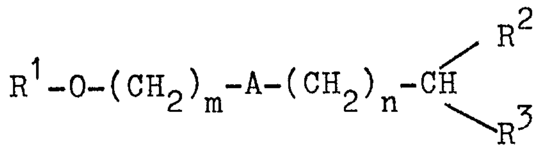

| Bsp. | $R^1$ | $R^2$ | $R^3$ | A | m | n | Salz | Schmp (°C) |
|---|---|---|---|---|---|---|---|---|
| 48 | 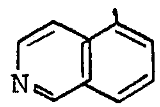 | 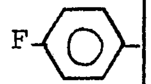 | 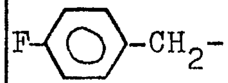 | 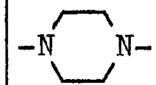 | 2 | 3 | Dimaleinat | 152 |
| 49 | 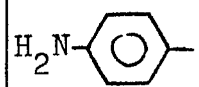 | " | 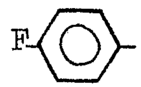 | " | 2 | 3 | Trihydro-chlorid | 173-177 (Z.) |
| 50 | 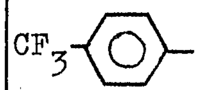 | " | " | " | 2 | 3 | Dihydro-chlorid | 196-199 |
| 51 | 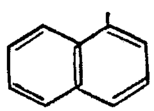 | " | " | 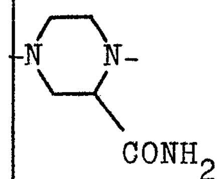 | 2 | 3 | Dihydro-chlorid | 140 (Z.) |
| 52 | " | " | 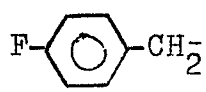 | 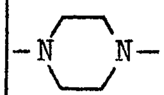 | 2 | 3 | Dimaleinat | 172-174 |
| 53 | 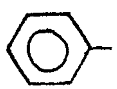 | 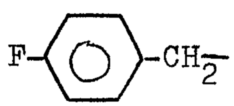 | 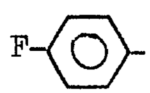 | " | 2 | 3 | " | 177-179 |

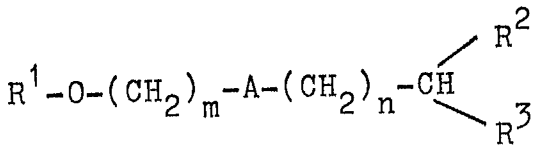

| Bsp. | $R^1$ | $R^2$ | $R^3$ | A | m | n | Salz | Schmp (°C) |
|---|---|---|---|---|---|---|---|---|
| 54 | 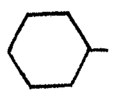 | 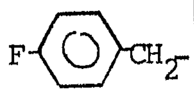 | 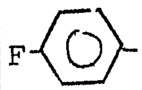 | 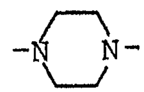 | 2 | 3 | Dimaleinat | 166-170 |
| 55 | 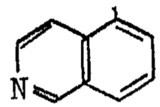 | 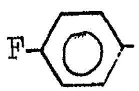 | " | 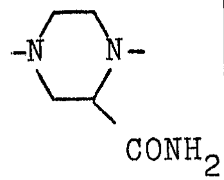 | 2 | 3 | Trihydro-chlorid | 170-174 |
| 56 | 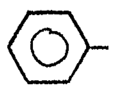 | " | 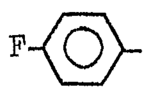 | " | 2 | 3 | Dihydro-chlorid | 118-122 |
| 57 | 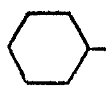 | " | " | " | 2 | 3 | " | 135-136 |
| 58 | 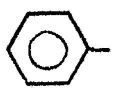 | " | " | 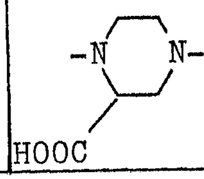 | 2 | 3 | " | 120-124 |

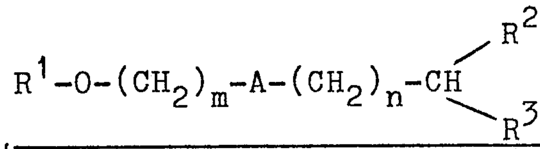

| Bsp. | $R^1$ | $R^2$ | $R^3$ | A | m | n | Salz | Schmp (°C) |
|---|---|---|---|---|---|---|---|---|
| 59 | 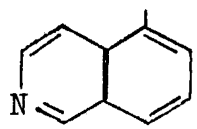 | 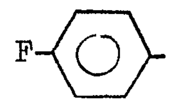 | 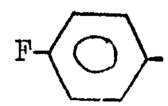 | 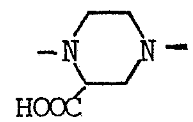 | 2 | 3 | Dihydro-chlorid | 173-178 |
| 60 | 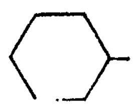 | " | " | " | 2 | 3 | " | 100-105 |
| 61 | 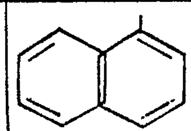 | " | " | " | 2 | 3 | " | Sintert ab 110 |
| 62 | 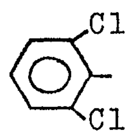 | " | " | 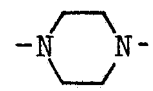 | 2 | 3 | " | 180-181 |
| 63 | 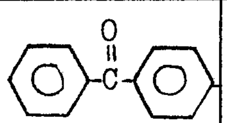 | " | " | " | 2 | 3 | " | 130-135 |
| 64 | 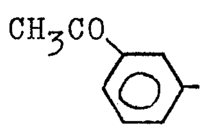 | " | " | " | 2 | 3 | " | 205-207 |

0229623

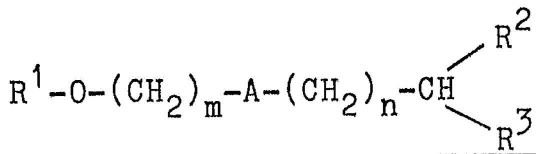

| Bsp. | R¹ | R² | R³ | A | m | n | Salz | Schmp (°C) |
|---|---|---|---|---|---|---|---|---|
| 65 | 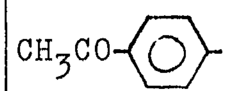 | 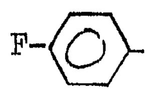 | 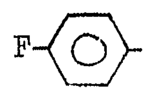 | 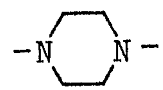 | 2 | 3 | Dihydro-chlorid | 159-161 |
| 66 | 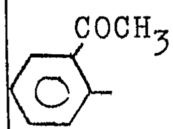 | " | " | " | 2 | 3 | " | 173-174 |
| 67 | 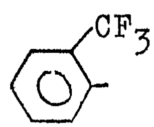 | " | " | " | 2 | 3 | " | 192-194 |
| 68 | 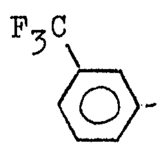 | " | " | " | 2 | 3 | " | 184-185 |
| 69 | 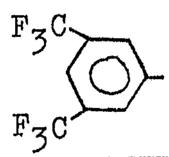 | " | " | " | 2 | 3 | " | 223-224 |

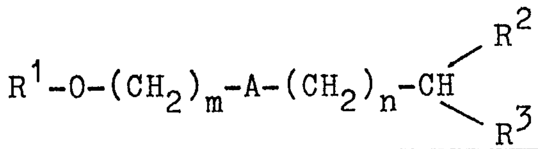

| Bsp. | $R^1$ | $R^2$ | $R^3$ | A | m | n | Salz | Schmp (°C) |
|---|---|---|---|---|---|---|---|---|
| 70 | 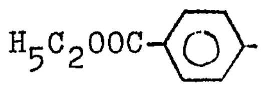 | 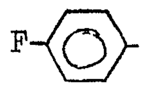 | 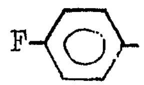 | 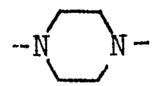 | 2 | 3 | Dihydro-chlorid | 160-165 |
| 71 | 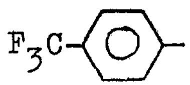 | " | " | 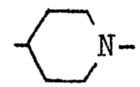 | 2 | 3 | - | ölig |
| 72 | 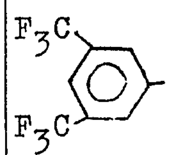 | " | " | " | 2 | 3 | - | " |
| 73 | 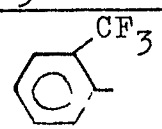 | " | " | " | 2 | 3 | - | " |
| 74 | 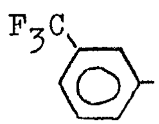 | " | " | " | 2 | 3 | " | " |

0229623

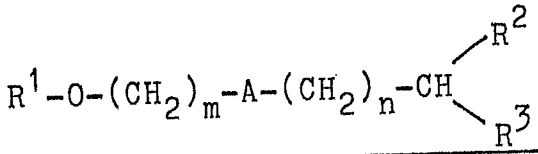

| Bsp. | $R^1$ | $R^2$ | $R^3$ | A | m | n | Salz | Schmp (°C) |
|---|---|---|---|---|---|---|---|---|
| 75 | 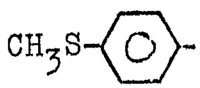 | 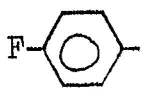 | 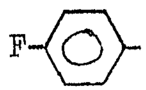 | 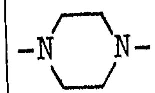 | 2 | 3 | Dihydro-chlorid | 208-211 |
| 76 | 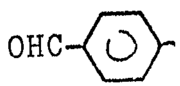 | " | " | " | 2 | 3 | " | 216-220 |
| 77 | 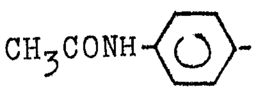 | " | " | " | 2 | 3 | Dioxalat | 228-235 (Zers.) |
| 78 | 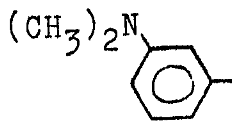 | " | " | " | 2 | 3 | Dimaleinat | 204-207 (Zers.) |
| 79 | 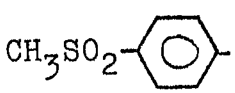 | " | " | " | 2 | 3 | Dihydro-chlorid | 232-238 |
| 80 | 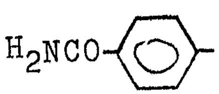 | " | " | " | 2 | 3 | " | 214-219 (Zers.) |

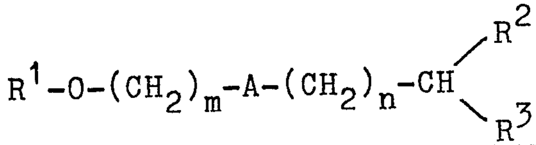

| Bsp. | $R^1$ | $R^2$ | $R^3$ | A | m | n | Salz | Schmp (°C) |
|---|---|---|---|---|---|---|---|---|
| 81 | 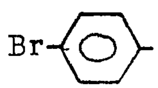 | 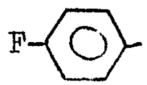 | 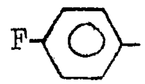 | 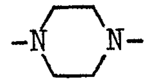 | 2 | 3 | Dihydro-chlorid | 215-218 |
| 82 | 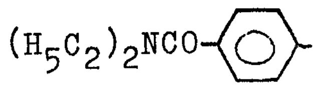 | " | " | " | 2 | 3 | Dimaleinat | 195-198 |
| 83 | 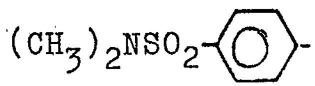 | " | " | " | 2 | 3 | Dihydro-chlorid | 219-222 |
| 84 | 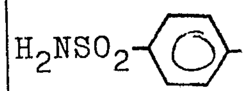 | " | " | " | 2 | 3 | Dimaleinat | 202-205 |
| 85 | 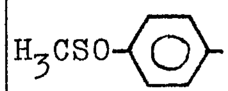 | " | " | " | 2 | 3 | " | 212-214 |
| 86 | 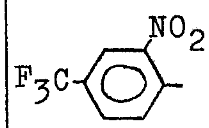 | " | " | " | 2 | 3 | Dihydro-chlorid | 158-160 |

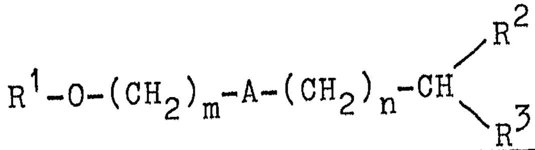

| Bsp. | $R^1$ | $R^2$ | $R^3$ | A | m | n | Salz | Schmp (°C) |
|---|---|---|---|---|---|---|---|---|
| 87 | 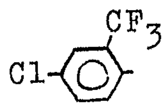 | 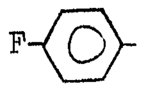 | 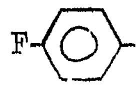 | 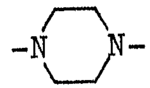 | 2 | 3 | Dihydro-chlorid | 215-219 |
| 88 | 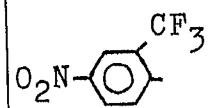 | " | " | " | 2 | 3 | " | 198-200 |
| 89 | 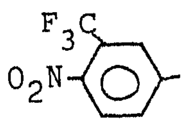 | " | " | " | 2 | 3 | Dimaleinat | 173-175 |
| 90 | 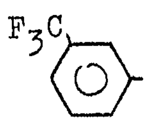 | 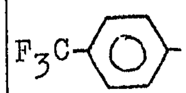 | 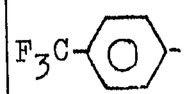 | " | 2 | 3 | " | 195-198 |
| 91 | 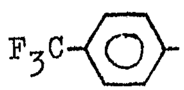 | " | " | " | 2 | 3 | " | 200-202 |
| 92 | " | 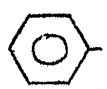 | 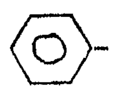 | " | 2 | 3 | Dihydro-chlorid | 226-230 |

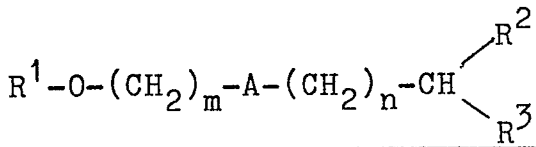

| Bsp. | $R^1$ | $R^2$ | $R^3$ | A | m | n | Salz | Schmp (°C) |
|---|---|---|---|---|---|---|---|---|
| 93 | 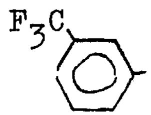 | 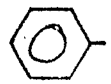 | 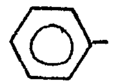 | 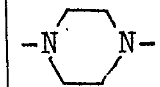 | 2 | 3 | Dihydro-chlorid | 218-220 |
| 94 | 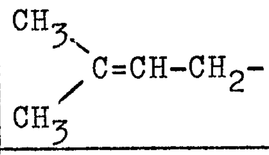 | 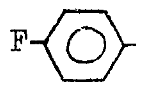 | 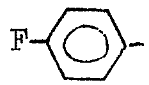 | " | 2 | 3 | " | 183-185 |
| 95 | 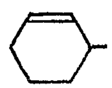 | " | " | " | 2 | 3 | " | 163-164 |

Beispiel 96: 1-[4,4-Bis-(4-fluorphenyl)butyl]-4-[2-(2,4-dinitrophenoxy)ethyl]piperazin

Zu einer Suspension von 0,432 g (10 mmol) 55% Natriumhydrid-Dispersion in 10 ml absolutem Dimethylformamid wurden 3,36 g (9,0 mmol) 1-[4,4-Bis-(4-fluorphenyl)-butyl]-4-(2-hydroxyethyl)piperazin, gelöst in 12 ml absolutem Dimethylformamid, innerhalb von 5 Minuten zugetropft und anschliessend jeweils eine Stunde bei 20°C und dann bei 40°C nachgerührt. Nun wurde unter Eiskühlung eine Lösung von 1,82 g (9,0 mmol) 2,4-Dinitrochlorbenzol in 10 ml absolutem Dimethylformamid so zugetropft, daß die Temperatur nicht über 10°C anstieg. Anschließend wurde noch 3 Stunden bei Zimmertemperatur gerührt, auf 300 ml Wasser gegeben und zweimal mit Methylenchlorid ausgeschüttelt. Die organischen Extrakte wurden vereinigt, getrocknet, am Ölpumpenvakuum bei 40°C eingeengt und durch Säulenchromatographie an Kieselgel mit $CH_2Cl_2/CH_3OH$ (99:1 bis 97,5:2,5) gereinigt.
Ausbeute: 2,50 g gelbliches Öl.
Zur Darstellung des Dihydrochlorids wurden 2,40 g der Base in 50 ml Isopropanol heiß gelöst und durch Zugabe von etherischer HCl auf pH 2 gestellt. Der beim Abkühlen aufgetretene Niederschlag wurde durch Zugabe von 100 ml Ether vervollständigt, abgesaugt und getrocknet.
Ausbeute: 2,40 g, Schmp. 183-185 °C.

Beispiel 97: 1-[4,4-Bis-(4-fluorphenyl)butyl]-4-[2-(2-methyl-4-nitrophenoxy)ethyl]piperazin

Zu einer Suspension von 0,43 g (10 mmol) 55% Natriumhydrid-Dispersion in 10 ml absolutem Dimethylformamid wurden 3,36 g (9,0 mmol) 1-[4,4-Bis-(4-fluorphenyl)-butyl]-4-(2-hydroxyethyl)piperazin, gelöst in 15 ml absolutem Dimethylformamid, zugetropft und 90 Minuten bei 60°C gerührt. Unter Eiskühlung wurde nun eine Lösung von 1,28 g (0,90 mol) 2-Fluor-5-nitrotoluol in 5 ml absolutem Dimethylformamid zugegeben und 3 Stunden bei Zimmertemperatur nachgerührt. Die Reaktionslösung wurde auf Wasser gegeben, zweimal mit

Methylenchlorid extrahiert und die vereinigten organischen Phasen getrocknet, eingeengt und durch Säulenchromatographie an Kieselgel mit Toluol/Ethanol (99:1 bis 95:5) gereinigt.
Ausbeute: 2,80 g farbloses Öl.

Zur Darstellung des Dihydrochlorids wurden 2,70 g der Base in 30 ml Essigester gelöst und mit etherischer HCl versetzt. Der ausgefallene Niederschlag wurde abgesaugt und getrocknet.
Ausbeute: 2,75 g; Schmp. 202-204°C.

Patentansprüche:

1. Verbindung der Formel I,

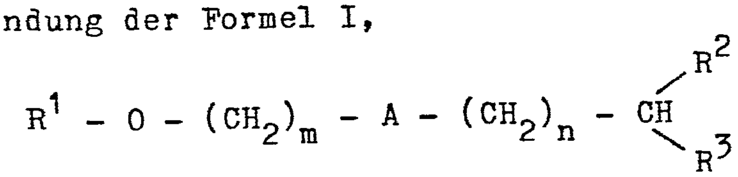 (I)

in welcher bedeuten:

$R^1$ $(C_3-C_8)$-Cycloalkyl, $(C_2-C_6)$-Alkenyl, geradkettig oder verzweigt, $(C_5-C_8)$-Cycloalkenyl,

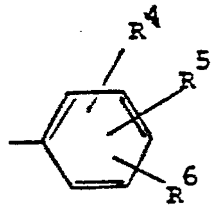 , 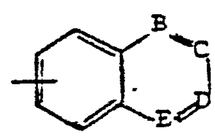 oder 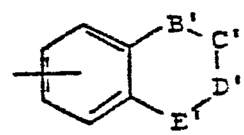

worin
$R^4$, $R^5$ und $R^6$ gleich oder verschieden unabhängig voneinander Wasserstoff, $(C_1-C_6)$-Alkyl, $(C_3-C_8)$-Cycloalkyl, Hydroxy, $(C_1-C_4)$-Alkoxy, $(C_1-C_4)$-Alkylthio, F, Cl, Br, J, Nitro, Cyano, Trifluormethyl, Formyl, Carboxyl, $(C_1-C_6)$-Alkoxycarbonyl, $(C_1-C_6)$-Acyl, Carbamoyl, N-Mono- oder N,N-Di$(C_1-C_6)$Alkylcarbamoyl, Sulfo, $(C_1-C_6)$-Alkoxysulfonyl, Sulfamoyl, N-Mono- oder N,N-Di-$(C_1-C_6)$-Alkylsulfamoyl, $(C_1-C_6)$-Alkylsulfinyl, $(C_1-C_6)$-Alkylsulfonyl, Amino, unsubstituiert oder substituiert mit ein oder zwei gleich- oder verschiedenartigen $(C_1-C_6)$-Alkyl-, $(C_1-C_6)$-Acyl- oder Aryl-, vorzugsweise Phenylgruppen,

B, C, D und E gleich oder verschieden unabhängig voneinander Methin oder Stickstoff,

B', C', D' und E' gleich oder verschieden unabhängig voneinander Methylen, Carbonyl, Imino, unsubstituiert oder am Stickstoff substituiert durch $(C_1-C_6)$-Alkyl, $(C_1-C_6)$-Acyl oder Aryl-, vorzugsweise Phenyl,

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl-, ($C_1$-$C_4$)-Alkoxy, F, Cl, Br, J, Cyano, Nitro oder Trifluormethyl substituiert ist,

A ein Amin 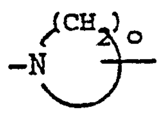 , 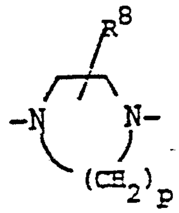 oder- 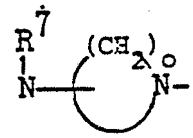

worin

$R^7$ Wasserstoff, ($C_1$-$C_6$)-Alkyl, Aryl, vorzugsweise Phenyl,

$R^8$ Wasserstoff, ($C_1$-$C_6$)-Alkyl, Formyl, ($C_1$-$C_6$)-Acyl, Carboxyl, ($C_1$-$C_6$)-Alkoxycarbonyl, Carbamoyl, N-Mono- oder N,N -Di-($C_1$-$C_6$)-Alkylcarbamoyl,

o 3, 4, 5, 6 oder 7,

p 2 oder 3,

m 2, 3 oder 4,

n 1, 2, 3 oder 4 bedeuten,

sowie die Salze der Verbindungen der Formel I mit physiologisch verträglichen Säuren.

2. Verbindung der Formel I nach Anspruch 1, in welcher mindestens einer der Reste und Indices folgende Bedeutung hat:

$R^1$ ($C_3$-$C_8$)-Cycloalkyl, 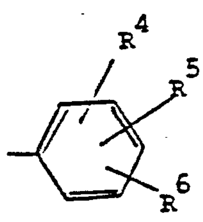 , 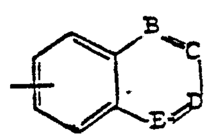

worin $R^4$ und $R^5$ gleich oder verschieden unabhängig voneinander Wasserstoff, ($C_1$-$C_6$)-Alkyl, ($C_1$-$C_4$)-Alkoxy, F, Cl, Br, J, Nitro, Cyano, Trifluormethyl, Formyl, Carboxyl, ($C_1$-$C_6$)-Alkoxycarbonyl, ($C_1$-$C_6$)-Acyl, Carbamoyl, N-Mono- oder N,N-Di-($C_1$-$C_6$)-Alkylcarbamoyl, Sulfo, ($C_1$-$C_6$)-Alkoxysulfonyl, Sulfamoyl, N-Mono- oder N,N-Di-($C_1$-

$C_6$)-Alkylsulfamoyl, ($C_1$-$C_6$)-Alkylsulfinyl, ($C_1$-$C_6$)-Alkylsulfonyl,

$R^6$ Wasserstoff,

B, C, D und E gleich oder verschieden unabhängig voneinander Methin oder Stickstoff,

$R^2$ und $R^3$ gleich oder verschieden und unabhängig voneinander Phenyl, das unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,

A ein Amin 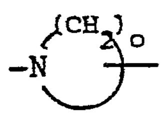 , 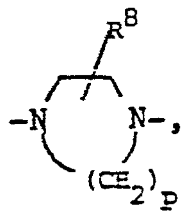 , 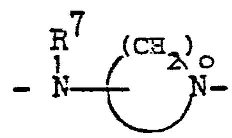

worin

$R^7$ Wasserstoff, Methyl, Ethyl,

$R^8$ Wasserstoff, Carboxyl, Carbamoyl,

o 4, 5 oder 6,

p 2 oder 3,

m 2, 3 oder 4,

n 1, 2, 3 oder 4 bedeuten.

3. Verbindung der Formel I nach Anspruch 1, in welcher mindestens einer der Substituenten und Indices folgende Bedeutung hat:

$R^1$ ($C_5$-$C_7$)-Cycloalkyl, 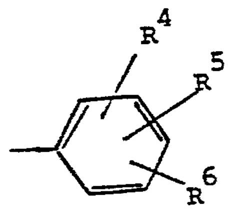 , 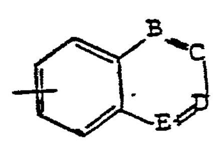

worin

$R^4$ Wasserstoff, Methyl, Ethyl, Propyl, Isopropyl, n-

Butyl, s-Butyl, Isobutyl, tert. Butyl, Methoxy, Ethoxy, Fluor, Chlor, Brom, Nitro, Cyano, Trifluormethyl,

$R^5$ und $R^6$ Wasserstoff,

B, C, D und E gleich oder verschieden unabhängig voneinander Methin oder Stickstoff,

$R^2$ und $R^3$ gleich oder verschieden unabhängig voneinander Phenyl, das unsubstituiert oder durch einen, zwei oder drei Substituenten aus der Gruppe Methyl, Fluor, Chlor, Brom, Cyano, Nitro oder Trifluormethyl substituiert ist,

A ein Amin 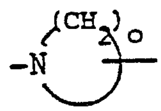 , 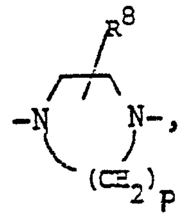

worin

$R^8$ Wasserstoff, Carboxyl, Carbamoyl,

o 4, 5 oder 6,

p 2 oder 3,

m 2, 3 oder 4,

n 2, 3 oder 4 bedeuten,

sowie die Salze der Verbindungen der Formel I mit physiologisch verträglichen Säuren.

4. Verbindung der Formel I nach Anspruch 1, in welcher mindestens einer der Substituenten oder Indices folgende Bedeutung hat:

$R^1$ Cyclohexyl, Phenyl, das unsubstituiert oder durch Methyl, tert. Butyl, Methoxy, Fluor, Nitro, Cyano oder Trifluormethyl substituiert ist, Naphthyl, Chinolinyl oder Isochinolinyl,

$R^2$ und $R^3$ gleich oder verschieden unabhängig voneinander Phenyl, das unsubstituiert oder durch Fluor oder Trifluormethyl substituiert ist,

A ein Amin 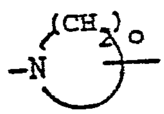 , 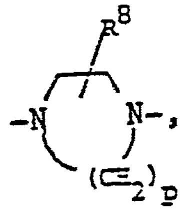

worin
$R^8$ Wasserstoff,
o 5,
p 2,
m 2,
n 3 bedeuten.

sowie die Salze der Verbindungen der Formel I mit physiologisch verträglichen Säuren.

5. Verfahren zur Herstellung von Verbindungen der Formel I nach Anspruch 1, dadurch gekennzeichnet, daß man

a) eine Verbindung der Formel II,

$$R^1 - O - (CH_2)_m - Y \qquad (II),$$

in welcher $R^1$ und m die gleiche Bedeutung wie in Formel I haben, und in welcher Y eine Abgangsgruppe, die nucleophil verdrängt werden kann, bedeutet, mit einer Verbindung der Formel III,

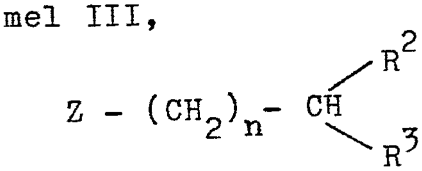 (III)

in welcher $R^2$, $R^3$ und n die gleiche Bedeutung wie in Formel I haben, und in welcher Z

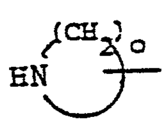 , 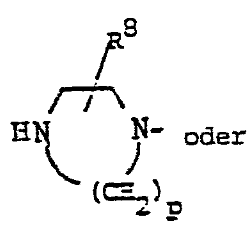 oder 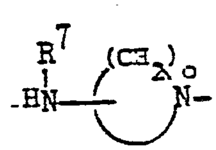

bedeutet, worin

$R^7$, $R^8$, o und p die gleiche Bedeutung wie in Formel I haben, unter Bedingungen einer nucleophilen Substitution umsetzt,

oder daß man

b) eine Verbindung der Formel IV,

$$R^1 - O - (CH_2)_m - Z \qquad (IV)$$

in welcher $R^1$ und m die gleiche Bedeutung wie in Formel I und Z die gleiche Bedeutung wie in Formel III haben, mit einer Verbindung der Formel V,

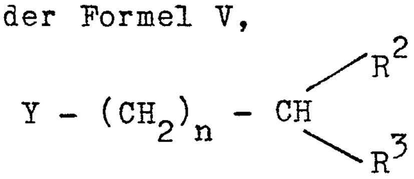

(V)

in welcher $R^2$, $R^3$ und n die gleiche Bedeutung wie in Formel I und Y die gleiche Bedeutung wie in Formel II haben,

unter den Bedingungen einer nucleophilen Substitution umsetzt,

oder daß man

c) eine Verbindung der Formel VI,

$$R^1 - OH \qquad (VI)$$

in der $R^1$ die gleiche Bedeutung wie in Formel I besitzt, mit einer Verbindung der Formel VII,

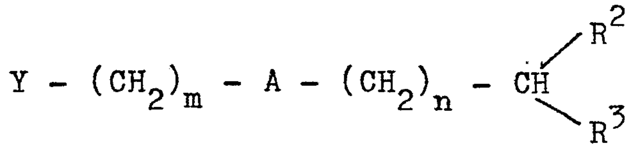

(VII)

in der $R^2$, $R^3$, A, m und n die gleiche Bedeutung wie in

Formel I und Y die gleiche Bedeutung wie in Formel II haben, in Gegenwart einer schwachen bis mittelstarken Base umsetzt, oder daß man

d) eine Verbindung der Formel XIII,

$R^1$-W (XIII)

in der $R^1$ die gleiche Bedeutung wie in Formel I besitzt und in der W eine Abgangsgruppe, die nucleophil verdrängt werden kann, bedeutet, mit einer Verbindung der Formel XIV,

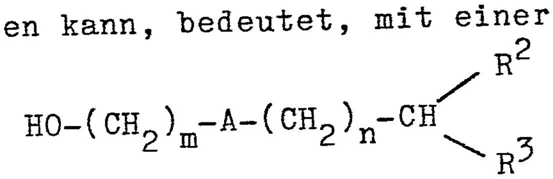

(XIV)

in der $R^2$, $R^3$, A, m und n die gleiche Bedeutung wie in Formel I besitzen, unter Bedingungen einer nucleophilen Substitution umsetzt.

6. Verbindung der Formel III

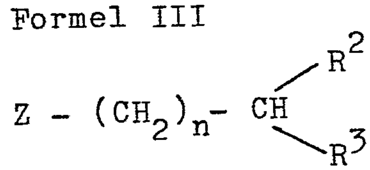

(III),

in welcher bedeuten:

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl, ($C_1$-$C_4$)-Alkoxy, F, Cl, Br, J, Cyano, Nitro oder Trifluormethyl substituiert ist,

Z 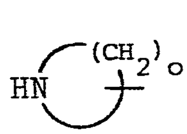 oder 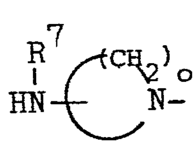 und

n 1, 2, 3 oder 4,

jedoch mit Ausnahme der Verbindung, in welcher gleichzeitig $R^2$=$R^3$=Phenyl, n=1 und Z = 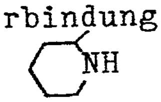 ist.

7. Verbindung der Formel VII

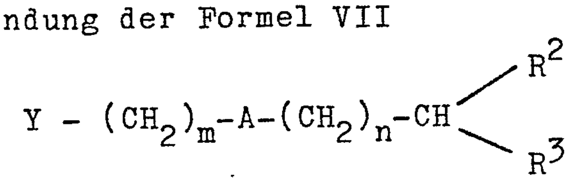 (VII)

in welcher bedeuten:

$R^2$ und $R^3$ gleich oder verschieden und unabhängig voneinander Phenyl, Phenyl-$(C_1-C_4)$-alkyl, wobei der Phenylring jeweils unsubstituiert oder durch einen, zwei oder drei Substituenten aus der Gruppe $(C_1-C_4)$-Alkyl-, $(C_1-C_4)$-Alkoxy, F, Cl, Br, J, Cyano, Nitro oder Trifluormethyl substituiert ist,

n 1, 2, 3 oder 4,

A ein Amin 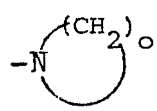 , 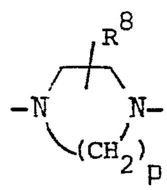 oder 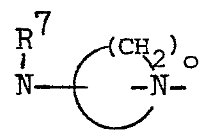

worin

$R^7$ Wasserstoff, $(C_1-C_6)$-Alkyl, Aryl, vorzugsweise Phenyl,

$R^8$ Wasserstoff, $(C_1-C_6)$-Alkyl, Formyl, $(C_1-C_6)$-Acyl, Carboxyl, $(C_1-C_6)$-Alkoxycarbonyl, Carbamoyl, N-Mono- oder N,N-Di-$(C_1-C_6)$-Alkylcarbamoyl,

o 3, 4, 5, 6 oder 7,

p 2 oder 3,

m 2, 3 oder 4,

Y eine Abgangsgruppe, die nucleophil verdrängt werden kann,

jedoch mit der Ausnahme der Verbindung, in welcher gleichzeitig

$R^2 = R^3$ = 4-Fluorphenyl,

n = 3

A = 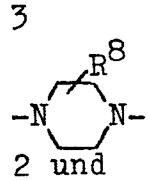 mit $R^8$ = H

m = 2 und

Y = Cl sind

und mit Ausnahme der Verbindung, in welcher gleichzeitig $R^2$=$R^3$=Phenyl, n=1, A = 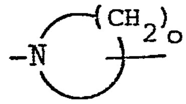 mit o=5, m=2 und Y=Cl sind.

8. Heilmittel zur Behandlung von Herz-Kreislauf- und cerebrovaskulären Erkrankungen, gekennzeichnet durch einen wirksamen Gehalt einer Verbindung I nach Anspruch 1.

9. Verwendung einer Verbindung I nach Anspruch 1 zur Herstellung eines Medikaments zur Behandlung von Herz-Kreislauf- und cerebrovaskulären Erkrankungen.

0229623
HOE 86/F 005

Patentansprüche Griechenland, Österreich und Spanien:

1. Verfahren zur Herstellung von Verbindungen der Formel I

$$R^1 - O - (CH_2)_m - A - (CH_2)_n - CH\begin{matrix} R^2 \\ R^3 \end{matrix} \qquad (I)$$

in welcher bedeuten:

$R^1$ $(C_3-C_8)$-Cycloalkyl, $(C_2-C_6)$-Alkenyl, geradkettig oder verzweigt, $(C_5-C_8)$-Cycloalkenyl,

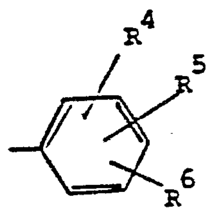 , 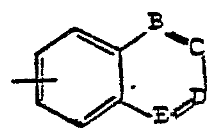 oder 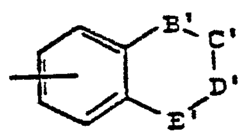

worin
$R^4$, $R^5$ und $R^6$ gleich oder verschieden unabhängig voneinander Wasserstoff, $(C_1-C_6)$-Alkyl, $(C_3-C_8)$-Cycloalkyl, Hydroxy, $(C_1-C_4)$-Alkoxy, $(C_1-C_4)$-Alkylthio, F, Cl, Br, J, Nitro, Cyano, Trifluormethyl, Formyl, Carboxyl, $(C_1-C_6)$-Alkoxycarbonyl, $(C_1-C_6)$-Acyl, Carbamoyl, N,N-Mono- oder Di-$(C_1-C_6)$-Alkylcarbamoyl, Sulfo, $(C_1-C_6)$-Alkoxysulfonyl, Sulfamoyl, N-Mono- oder N,N-Di-$(C_1-C_6)$-Alkylsulfamoyl, $(C_1-C_6)$-Alkylsulfinyl, $(C_1-C_6)$-Alkylsulfonyl, Amino, unsubstituiert oder substituiert mit ein oder zwei gleich- oder verschiedenartigen $(C_1-C_6)$-Alkyl-, $(C_1-C_6)$-Acyl- oder Aryl-, vorzugsweise Phenylgruppen,

B, C, D und E gleich oder verschieden unabhängig voneinander Methin oder Stickstoff,

B', C', D' und E' gleich oder verschieden unabhängig voneinander Methylen, Carbonyl, Imino, unsubstituiert oder am Stickstoff substituiert durch $(C_1-C_6)$-Alkyl, $(C_1-C_6)$-Acyl oder Aryl-, vorzugsweise Phenyl,

$R^2$ und $R^3$ gleich oder verschieden und unabhängig voneinander Phenyl, Phenyl-($C_1$-$C_4$)-alkyl, wobei der Phenylring jeweils unsubstituiert oder durch einen, zwei oder drei Substituenten aus der Gruppe ($C_1$-$C_4$)-Alkyl, ($C_1$-$C_4$)-Alkoxy, F, Cl, Br, J, Cyano, Nitro oder Trifluormethyl substituiert ist,

A ein Amin 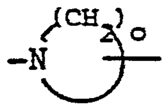 , 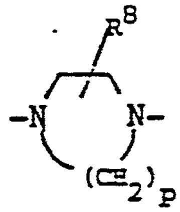 oder 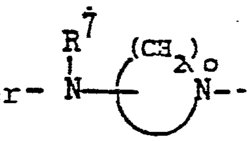

worin

$R^7$ Wasserstoff, ($C_1$-$C_6$)-Alkyl, Aryl, vorzugsweise Phenyl,

$R^8$ Wasserstoff, ($C_1$-$C_6$)-Alkyl, Formyl, ($C_1$-$C_6$)-Acyl, Carboxyl, ($C_1$-$C_6$)-Alkoxycarbonyl, Carbamoyl, N-Mono- oder N,N'-Di-($C_1$-$C_6$)-Alkylcarbamoyl,

o 3, 4, 5, 6 oder 7,

p 2 oder 3,

m 2, 3 oder 4,

n 1, 2, 3 oder 4 bedeuten,

sowie die Salze der Verbindungen der Formel I mit physiologisch verträglichen Säuren, dadurch gekennzeichnet, daß man

a) eine Verbindung der Formel II,

$$R^1 - O - (CH_2)_m - Y \qquad (II),$$

in welcher $R^1$ und m die gleiche Bedeutung wie in Formel I haben, und in welcher Y eine Abgangsgruppe, die nucleophil verdrängt werden kann, bedeutet,
mit einer Verbindung der Formel III,

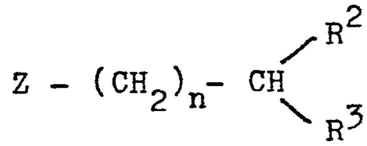 (III)

in welcher $R^2$, $R^3$ und n die gleiche Bedeutung wie in

Formel I haben, und in welcher Z

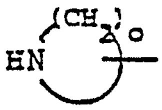 , 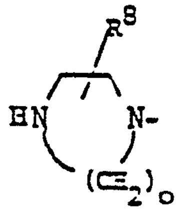 oder 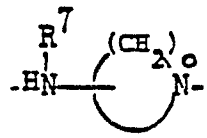

bedeutet, worin

$R^7$, $R^8$, o und p die gleiche Bedeutung wie in Formel I haben,

unter Bedingungen einer nucleophilen Substitution umsetzt,

oder daß man

b) eine Verbindung der Formel IV,

$$R^1 - O - (CH_2)_m - Z \qquad (IV)$$

in welcher $R^1$ und m die gleiche Bedeutung wie in Formel I und Z die gleiche Bedeutung wie in Formel III haben, mit einer Verbindung der Formel V,

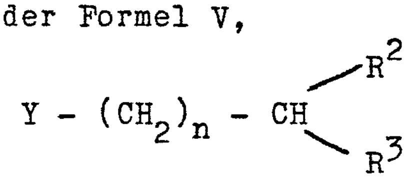 (V)

in welcher $R^2$, $R^3$ und n die gleiche Bedeutung wie in Formel I und Y die gleiche Bedeutung wie in Formel II haben,

unter den Bedingungen einer nucleophilen Substitution, umsetzt,

oder daß man

c) eine Verbindung der Formel VI,

$$R^1 - OH \qquad (VI)$$

in der $R^1$ die gleiche Bedeutung wie in Formel I besitzt, mit einer Verbindung der Formel VII,

$$Y - (CH_2)_m - A - (CH_2)_n - CH\begin{matrix} R^2 \\ R^3 \end{matrix} \qquad (VII)$$

in der $R^2$, $R^3$, A, m und n die gleiche Bedeutung wie in Formel I und Y die gleiche Bedeutung wie in Formel II haben, in Gegenwart einer schwachen bis mittelstarken Base umsetzt, oder daß man

d) eine Verbindung der Formel XIII,

$$R^1-W \qquad (XIII)$$

in der $R^1$ die gleiche Bedeutung wie in Formel I besitzt und in der W eine Abgangsgruppe, die nucleophil verdrängt werden kann, bedeutet, mit einer Verbindung der Formel XIV,

$$HO-(CH_2)_m-A-(CH_2)_n-CH\begin{matrix} R^2 \\ R^3 \end{matrix} \qquad (XIV)$$

in der $R^2$, $R^3$, A, m und n die gleiche Bedeutung wie in Formel I besitzen, unter Bedingungen einer nucleophilen Substitution umsetzt.

2. Verfahren zum Herstellen einer Verbindung I nach Anspruch 1, dadurch gekennzeichnet, daß mindestens einer der Reste und Indices folgende Bedeutung hat:

$R^1$ $(C_3-C_8)$-Cycloalkyl, 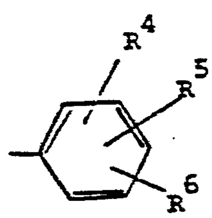 , 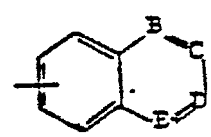

worin $R^4$ und $R^5$ gleich oder verschieden unabhängig voneinander Wasserstoff, $(C_1-C_6)$-Alkyl, $(C_1-C_4)$-Alkoxy, F, Cl, Br, J, Nitro, Cyano, Trifluormethyl, Formyl, Carboxyl, $(C_1-C_6)$-Alkoxycarbonyl, $(C_1-C_6)$-Acyl, Carbamoyl, N-Mono- oder N,N-Di-$(C_1-C_6)$-Alkylcarbamoyl, Sulfo, $(C_1-C_6)$-Alkoxysulfonyl, Sulfamoyl, N-Mono- oder N,N-Di-$(C_1-$

$C_6$)-Alkylsulfamoyl, ($C_1$-$C_6$)-Alkylsulfinyl, ($C_1$-$C_6$)-Alkylsulfonyl,

$R^6$ Wasserstoff,

B, C, D und E gleich oder verschieden unabhängig voneinander Methin oder Stickstoff,

$R^2$ und $R^3$ gleich oder verschieden und unabhängig voneinander Phenyl, das unsubstituiert oder durch einen, zwei oder drei Substituenten aus der Gruppe Methyl, Ethyl, Methoxy, Ethoxy, F, Cl, Br, J, Cyano, Nitro oder Trifluormethyl substituiert ist,

A ein Amin 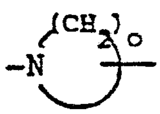 , 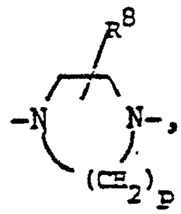 , 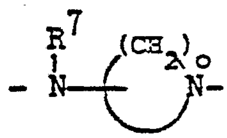

worin
$R^7$ Wasserstoff, Methyl, Ethyl,
$R^8$ Wasserstoff, Carboxyl, Carbamoyl,
o 4, 5 oder 6,
p 2 oder 3,
m 2, 3 oder 4,
n 1, 2, 3 oder 4 bedeuten.

3. Verfahren zum Herstellen einer Verbindung I nach Anspruch 1, in welchem mindestens einer der Substituenten und Indices folgende Bedeutung hat:

$R^1$ ($C_5$-$C_7$)-Cycloalkyl, 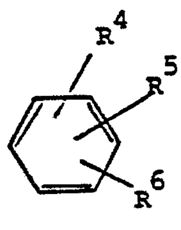 , 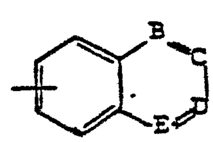

worin

$R^4$ Wasserstoff, Methyl, Ethyl, Propyl, Isopropyl, n-

Butyl, s-Butyl, Isobutyl, tert. Butyl, Methoxy, Ethoxy, Fluor, Chlor, Brom, Nitro, Cyano, Trifluormethyl,
$R^5$ und $R^6$ Wasserstoff,

B, C, D und E gleich oder verschieden unabhängig voneinander Methin oder Stickstoff,

$R^2$ und $R^3$ gleich oder verschieden unabhängig voneinander Phenyl, das unsubstituiert oder durch einen, zwei oder drei Substituenten aus der Gruppe Methyl, Fluor, Chlor, Brom, Cyano, Nitro oder Trifluormethyl substituiert ist,

A ein Amin

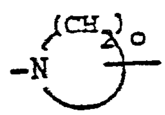
,
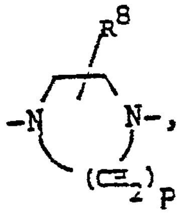
,

worin
$R^8$ Wasserstoff, Carboxyl, Carbamoyl,
o 4, 5 oder 6,
p 2 oder 3,
m 2, 3 oder 4
n 2, 3 oder 4 bedeuten.

4. Verfahren zum Herstellen einer Verbindung I nach Anspruch 1, in welchem mindestens einer der Substituenten oder Indices folgende Bedeutung hat:

$R^1$ Cyclohexyl, Phenyl, das unsubstituiert oder durch Methyl, tert. Butyl, Methoxy, Fluor, Nitro, Cyano oder Trifluormethyl substituiert ist, Naphthyl, Chinolinyl oder Isochinolinyl,

$R^2$ und $R^3$ gleich oder verschieden unabhängig voneinander Phenyl, das unsubstituiert oder durch Fluor oder Trifluormethyl substituiert ist,

A ein Amin

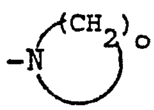 , 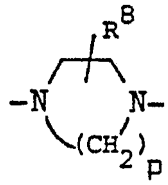

worin

$R^8$ Wasserstoff,

o 5,

p 2,

m 2,

n 3 bedeuten.

5. Verfahren zum Herstellen einer Verbindung der Formel VII

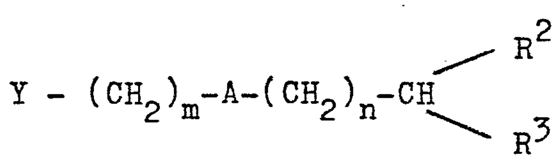 (VII)

in welcher bedeuten:

$R^2$ und $R^3$ gleich oder verschieden und unabhängig voneinander Phenyl, Phenyl-$(C_1-C_4)$-alkyl, wobei der Phenylring jeweils unsubstituiert oder durch einen, zwei oder drei Substituenten aus der Gruppe $(C_1-C_4)$-Alkyl, $(C_1-C_4)$-Alkoxy, F, Cl, Br, J, Cyano, Nitro oder Trifluormethyl substituiert ist,

n 1, 2, 3 oder 4,

A ein Amin 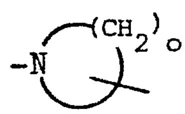, 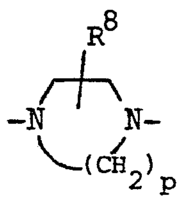 oder 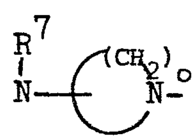

worin

$R^7$ Wasserstoff, $(C_1-C_6)$-Alkyl, Aryl, vorzugsweise Phenyl,

$R^8$ Wasserstoff, $(C_1-C_6)$-Alkyl, Formyl, $(C_1-C_6)$-Acyl, Carboxyl, $(C_1-C_6)$-Alkoxycarbonyl, Carbamoyl, N-Mono- oder N,N-Di-$(C_1-C_6)$-Alkylcarbamoyl,

o 3, 4, 5, 6 oder 7,

p 2 oder 3,

 

0229623

m 2, 3 oder 4,

Y eine Abgangsgruppe, die nucleophil verdrängt werden kann,

jedoch mit der Ausnahme der Verbindung, in welcher gleichzeitig

$R^2 = R^3$ = 4-Fluorphenyl,

n = 3

A = 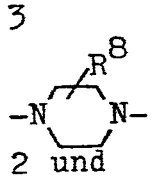 mit $R^8$ = H

m = 2 und

Y = Cl sind

und mit Ausnahme der Verbindung, in welcher gleichzeitig $R^2$=$R^3$=Phenyl, n=1, A = 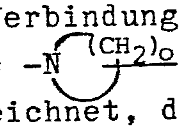 mit o=5, m=2 und Y=Cl sind, dadurch gekennzeichnet, daß man eine Verbindung der Formel V

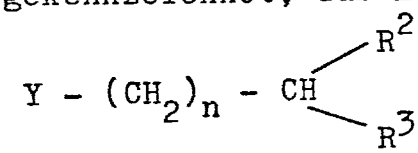 (V)

in welcher $R^2$, $R^3$ und n die gleiche Bedeutung wie in Formel I und Y die gleiche Bedeutung wie in Formel II haben, mit einer Verbindung der Formel XII,

$$HO - (CH_2)_m - Z \qquad (XII)$$

worin m die gleiche Bedeutung wie in Formel I und Z die gleiche Bedeutung wie in Formel III besitzen, unter den Bedingungen einer nucleophilen Substitution umsetzt und anschließend die Hydroxyfunktion in die Abgangsgruppe Y nach gängigen Methoden überführt.

6. Verfahren zum Herstellen einer Verbindung VII nach Anspruch 5, dadurch gekennzeichnet, daß man eine Verbindung der Formel V

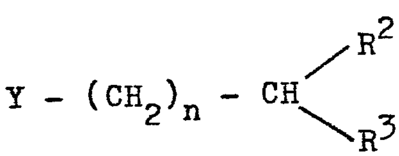 (V)

in welcher $R^2$, $R^3$ und n die gleiche Bedeutung wie in

Formel I und Y die gleiche Bedeutung wie in Formel II haben, mit Verbindung der Formel XI,

$$Y - (CH_2)_m - Z \qquad (XI)$$

worin m die gleiche Bedeutung wie in Formel I, Y die gleiche Bedeutung wie in Formel II und Z die gleiche Bedeutung wie in Formel III besitzen, unter den Bedingungen einer nucleophilen Substitution umsetzt.

7. Verfahren zum Herstellen eines Medikaments zur Behandlung von Herz-Kreislauf- und cerebrovaskulären Erkrankungen, dadurch gekennzeichnet, daß man eine wirksame Menge einer Verbindung I nach Anspruch 1 beziehungsweise eines Salzes davon mit üblichen pharmazeutisch verträglichen Zusatzstoffen mischt.

8. Verwendung einer Verbindung I nach Anspruch 1 bzw. eines ihrer Salze zur Herstellung eines Medikaments zur Behandlung von Herz-Kreislauf- und cerebrovaskulären Erkrankungen.